# FASCICULE DE BREVET EUROPEEN

(11) **EP 1 876 232 B1**
(45) Date de publication et mention de la délivrance du brevet: **14.11.2018**
(21) Numéro de dépôt: 07368008.4
(22) Date de dépôt: 03.07.2007
(51) Int. Cl.: C12N 1/14, A01N 63/04, C12R 1/885

(54) **NOUVELLE SOUCHE DE TRICHODERMA ATROVIRIDE, UN MILIEU DE CULTURE LA CONTENANT, AINSI QUE L'UTILISATION DE LADITE SOUCHE NOTAMMENT COMME STIMULANT DE LA GERMINATION ET/OU DE LA CROISSANCE DES PLANTES**
NEUER STAMM VON TRICHODERMA ATROVIRIDE, EIN DIESEN STAMM ENTHALTENDES KULTURMILIEU SOWIE DIE VERWENDUNG DIESES STAMMS INSBESONDERE ALS STIMULANS ZUR KEIMUNG UND/ODER ZUM WACHSTUM VON PFLANZEN
NEW TRICHODERMA ATROVIRIDE STRAIN, CULTURE MEDIUM CONTAINING IT, AND USE OF THE STRAIN IN PARTICULAR AS A STIMULANT FOR THE GERMINATION AND/OR GROWTH OF PLANTS

(30) Priorité: 04.07.2006 FR 0606072
(43) Date de publication de la demande: 09.01.2008
(73) Titulaire: Nixe, 06905 Sophia Antipolis Cedex (FR)
(72) Inventeur: Canaguier, Renaud, 06130 Grasse (FR); Braquet, Sonia, 06200 Nice (FR); De Raco, Christine, 06550 La Roquette sur Siagne (FR)
(74) Mandataire: Murgitroyd & Company

(56) Documents cités:
- JP-A- 2006 124 280
- US-A1- 2004 067 851
- MCBEATH J H ET AL: "BIOLOGICAL CONTROL OF PINK ROT BY TRICHODERMA ATROVIRIDE" PHYTOPATHOLOGY, ST. PAUL, MN, US, vol. 91, no. 6, SUPPL, 25 août 2001 (2001-08-25), page S59, XP001026341 ISSN: 0031-949X
- KEXIANG G ET AL: "Potential of Trichoderma harzianum and T. atroviride to control Botryosphaeria berengeriana f. sp. piricola, the cause of apple ring rot" JOURNAL OF PHYTOPATHOLOGY (BERLIN), vol. 150, no. 4-5, mai 2002 (2002-05), pages 271-276, XP002416681 ISSN: 0931-1785
- GRAVEL V ET AL: "Microorganisms stimulating growth and development of greenhouse tomato plants." CANADIAN JOURNAL OF PLANT PATHOLOGY, vol. 27, no. 3, juillet 2005 (2005-07), page 469, XP008074091 & JOINT MEETING OF THE CANADIAN-PHYTOPATHOLOGICAL-SOCIETY/AMERICA N-PHYT OPATHOLOGICAL-SOCIETY; WINDSOR, CANADA; JUNE 29 -JULY 01, 2005 ISSN: 0706-0661
- GRAVEL V ET AL: "Potential biocontrol agents against Pythium root rot of greenhouse tomato." CANADIAN JOURNAL OF PLANT PATHOLOGY, vol. 27, no. 3, juillet 2005 (2005-07), page 469, XP008074092 & JOINT MEETING OF THE CANADIAN-PHYTOPATHOLOGICAL-SOCIETY/AMERICA N-PHYT OPATHOLOGICAL-SOCIETY; WINDSOR, CANADA; JUNE 29 -JULY 01, 2005 ISSN: 0706-0661
- "Safety data sheet TIFI"[Online] 15 mars 2006 (2006-03-15), XP002453590 Extrait de l'Internet: URL:http://www.giten.fr/pls/pazitalpo/pk$g es_upload.DOWNLOAD?nome_file=F7817/TIFI%20 SDS%20English.pdf> [extrait le 2007-10-03]
- WATANABE SATOSHI ET AL: "Properties and biological control activities of aerial and submerged spores in Trichoderma asperellum SKT-1" JOURNAL OF PESTICIDE SCIENCE, vol. 31, no. 4, septembre 2006 (2006-09), pages 375-379, XP002416682 ISSN: 1348-589X

## Description

La présente invention concerne essentiellement une nouvelle souche de *Trichoderma atroviride,* un ou des clones issus de cette nouvelle souche, un ascomycète possédant les caractéristiques de cette nouvelle souche, un milieu de culture, un concentré déshydraté, une poudre à base de ce nouveau *Trichoderma atroviride,* les utilisations de ce dernier, en particulier comme stimulateur de la germination et/ou de la croissance des plantes et/ou comme agent de biocontrôle, ainsi que son procédé d'isolement.

Le genre *Trichoderma* est un groupe agronomiquement important car il comprend des champignons agents de biocontrôle dont le mode d'action a fait l'objet de nombreux travaux. Des études récentes font en effet état d'une capacité de ce champignon filamenteux à intervenir selon divers mécanismes : mycoparasitisme, antagonisme (compétition), antibiose (production d'antibiotiques), stimulation racinaire, stimulation de la croissance par solubilisation de minéraux fertilisants, stimulation des défenses naturelles des plantes, etc.

Les champignons du genre *Trichoderma* appartiennent au phylum Ascomycota, classe des Ascomycetes, famille des Hypocreales. Ce sont des champignons microscopiques dont il existe des espèces terrestres et des espèces marines. On en trouve dans les bois en décomposition, les résidus végétaux et dans tous les sols (humus forestiers, terres agricoles) (de 10 à 10000 propagules/g dans les sols tempérés ou tropicaux). Ils colonisent les racines des plantes herbacées et ligneuses sans aucun dommage. En outre, ce champignon peut pénétrer dans les racines et favoriser le développement, la nutrition et la résistance aux maladies. L'espèce *atroviride* est l'une des espèces courantes de *Trichoderma.*

La colonisation des racines par *Trichoderma* sp. peut augmenter la croissance et le développement des racines, le rendement des cultures, la résistance aux stress abiotiques et l'absorption et l'utilisation des nutriments (Harman G.E. et al., Nature Reviews / Microbiology, 2, 43-56, 2004).

Lorsqu'ils existent, les effets de stimulation de croissance proviennent de l'action directe des *Trichoderma* sur les plantes et ne sont pas directement liés aux antagonismes avec les pathogènes. Ces effets sont visibles aussi bien sur des substrats de culture non désinfectés que sur des substrats stériles. Les mécanismes de stimulation de croissance sont mal élucidés et pourraient être dus à la suppression des dommages oxydatifs sur les racines, à la sécrétion de facteurs de croissance par le champignon, à l'inhibition de la microflore gênante et à l'amélioration du transport des micronutriments.

Les effets sont inégaux d'une souche à l'autre. Certaines souches possèdent des effets stimulateurs de croissance mais d'autres ont des effets inhibiteurs (par exemple, *Trichoderma viride* RF1, J.G. Menzies, Plant Pathology, 42, 784-791, 1993).

Les brevets FR2700542 et FR2780972 divulguent différentes alkyl-lactones produites par les souches de *Trichoderma harzianum* I-1235 et I-1536 pour le premier brevet et I-1571 pour le second et capables de stimuler la germination et la croissance. Le brevet US6753295 présente un mélange de peptaibols et d'alkyl-cyclopenténones produits par le *T. harzianum* SK-5-5 (NIBH 13327) stimulant la croissance et la défense des plantes. Le recours à la fusion de protoplastes de 2 souches mutantes a permis à A. Sivan et G.E. Harman (J. Gen. Microbiol., 137, 23-29, 1991) de produire une nouvelle souche de *Trichoderma harzianum* 1295-22 (ATCC 20847) dont la compétence avec la rhizosphère est améliorée et qui favorise la croissance du maïs de 66 % par rapport au témoin non traité (Björkman et al., J. Am. Soc. Hort., 123, 35-40, 1998).

Les propriétés antagonistes des *Trichoderma* sont mieux documentées que les propriétés stimulatrices. Le potentiel antagoniste des *Trichoderma* vis-à-vis de nombreux champignons du sol pathogènes des plantes a été découvert dans les années 1930 (Weindling R ., « Trichoderma lignorum as a parasite of other soil fungi », Phytopathology, 22, 837-845, 1932) . L'application la plus évidente est la lutte biologique en agriculture (biocontrôle), y compris en agriculture biologique où le règlement (CE) n° 2092/91 prévoit cette utilisation.

Les chercheurs ont remarqué très tôt que les propriétés antagonistes varient significativement d'une espèce à l'autre, et au sein d'une même espèce, d'une variété à l'autre.

L'usage de *Trichoderma* comme antifongique en agriculture a commencé à se développer en 1976 avec la lutte contre *Botrytis cinerea* de la vigne (SEPPIC FR2345921 (30/03/1976), *Trichoderma viride* Bisby). Par la suite, sont apparues des applications contre de nombreux autres champignons pathogènes tels *Armillaria mellea*, *Stereum purpureum*, *Ceratocystis ulmi*, *Pythium ultimum* et *Sclerotinia sclerotiorum* et *Eutypa lata* :
- Ricard J. GB1573850 (12/09/1977), souche I-030, *Trichoderma viride* Bisby, propriétés antifongiques contre diverses maladies, y compris *B. cinerea*,
- Papavizas G. US4489161 (25/10/1982), souche T-1-R9, *T. viride* Pers. ex S.F. Gray, agent de biocontrôle de *Fusarium oxysporum* du chrysanthème,
- Merlier O. *et al*. - ORSAN FR2545099 (28/4/1983), souche I-223 / ATCC 20672, *Trichoderma harzianum* Rifai, usage de la souche ou de peptides (trichorzianines) produits par la souche pour lutter contre *Botrytis cinerea*, *Stereum purpureum*, *Ceratocystis ulmi* et *Sclerotinia*,
- Harman G. EP0285987, souche 1295-22 (T-22) / ATCC 20847, *Trichoderma* sp., souche obtenue par fusion des protoplastes destinée à la protection des semences contre les maladies du sol,
- Speakman J.B. *et al*. - BASF EP0383201 (16/02/1989), souche 35/84 / IMI 311756 / DSMZ 5067, *Trichoderma* sp., fongicide pour les semences en particulier contre *Pythium*
- Elad Y. - PERI DEVELOPMENT APPLICATION EP0466133 (12/07/1990), souche T-39 / I-952, *Trichoderma harzianum,* antagoniste de *Botrytis cinerea* et *Sclerotinia sclerotiorum* compatible avec plusieurs fongicides chimiques
- Monte Vasquez E. - Universidad de Salamanca WO9716974 (07/11/1995), souches IMI 352940, IMI 352941, CECT 20179 et CECT 20178, respectivement trois *T. harzianum* Rifai et un *T. viride* Pers., agents de biocontrôle contre les maladies des plantes
- Besnard O. - B2B WO2005068609 (07/01/2004), souches I-1571 et I-3151, deux *T. harzianum* Rifai, agents de lutte contre les maladies du bois de la vigne en particulier Eutypiose et ESCA

Les espèces les plus fréquemment utilisées comme agents de biocontrôle, sont *T*. *harzianum* Rifai, *T. viride* Persoon et *Trichoderma polysporum*.

La capacité de *T. harzianum* à produire des métabolites antifongiques, y compris des composés volatils, explique bien cet usage. Les chitinases, glucanases et protéases libérées par ces *Trichoderma* dégradent les parois des champignons pathogènes sans altérer les cellules des racines des plantes. Les métabolites antibiotiques, tels les peptaibols (par exemple, trichorzianines), agissent en synergie avec les enzymes pour attaquer les champignons cibles. *T. harzianum* se développe au contact des racines et les colonise superficiellement sans aucun dommage.

Il a été montré que *Trichoderma virens* colonise les racines du coton et inhibe la croissance de *Macrophomina phaseolina* (pourriture des racines) apporté par le sol (Howell C.R., Plant disease, 87, 4-10, 2003). Le mycoparasitisme de *Pythium ultimum* par *T. atroviride* est un phénomène actif qui se produit directement sur la surface des graines et des racines de concombre. Le mycoparasitisme est un principe essentiel du biocontrôle qui n'exclut pas la participation de la compétition trophique, de l'occupation de l'espace, de l'antibiose et de la stimulation des défenses de la plante (Lu Z. et al., Appl. Environ. Microbiol., 70, 3073-3081, 2004).

La capacité particulière des *Trichoderma* à détruire des substances toxiques a été signalée et l'usage de *Trichoderma* en bioremédiation a été proposé (*cf.* Lynch J. WO03045596 (23/11/2001), *T. harzianum* pour la bioremédiation des sols contaminés par le cyanure et les hydrocarbures). On rappelle, à titre informatif, que la bioremédiation est un ensemble de techniques consistant à augmenter la biodégradation ou la biotransformation, en inoculant des microorganismes spécifiques (bioaugmentation) ou en stimulant l'activité de populations microbiennes indigènes (biostimulation) par apport de nutriments et par ajustement des conditions de milieu (potentiel d'oxydoréduction, humidité). Cette capacité particulière de destruction des substances toxiques, permet aux *Trichoderma* de détruire les toxines secrétées par les champignons et ainsi protéger les plantes des attaques de champignons phytopathogènes. L'eutypine, le 4-hydroxybenzaldehyde et l'acide 3-phenyllactique sont des phytotoxines produites par les champignons qui provoquent l'eutypiose et l'ESCA, maladies du bois de la vigne. Les *Trichoderma* peuvent dégrader ces toxines, et certaines souches les métabolisent complètement (Christen D. et al., J. Agric. Food Chem., 53, 7043-7051, 2005). Les *Trichoderma* peuvent résister aux toxiques et plus particulièrement aux antifongiques en dénaturant les molécules par oxydation.

La protection des plantes par les *Trichoderma* sp. s'établit au travers de nombreux mécanismes. Tous les *Trichoderma* ne sont pas équivalents car un bon agent de lutte biologique doit être un bon colonisateur et un bon antagoniste mais il doit également être compatible avec la rhizosphère et avec les plantes.

Il est apparu de manière surprenante que la souche de *Trichoderma atroviride* qui a été isolée et purifiée selon l'invention possède d'excellentes propriétés stimulatrices de croissance, des propriétés antagonistes utiles en agriculture ainsi qu'une grande stabilité en matière de viabilité.

La souche et les clones de *Trichoderma atroviride* isolés et purifiés par le déposant, ont été déposés auprès des Collections Coordonnées Belges de Microorganismes (BCCM™)/Mycothèque de l'Unversité catholique de Louvain (MUCL), le 3 mai 2004, sous le numéro BCCM™/MUCL 45632 ; cet organisme étant reconnu autorité de dépôt internationale au sens du Traité de Budapest. Cette nouvelle souche sera dénommée, ci-après, *Trichoderma atroviride* MUCL 45632 ou, plus simplement, MUCL 45632. Ladite souche possède le profil biochimique, morphologique et physiologique suivant :

| Milieu de culture | Vitesse de croissance | Aspect de la sporulation |
|---|---|---|
| (1) milieu de référence PDA à 25°C potato dextrose agar 39 g/L | très rapide - apparition d'un thalle ras translucide en deux jours environ | vert intense |

| Milieu de culture | Vitesse de croissance par rapport au milieu PDA | Aspect de la sporulation par rapport au milieu PDA |
|---|---|---|
| (2) milieu minimum à 4°C glucose 5 g/L + peptone 5 g/L + Mg SO₄, 7 H₂O 0,5 g/L + KH₂PO₄ 1 g/L + rose bengale 25 mg/L + agar 15 g/L | très lente | absente |
| (3) milieu minimum à 25°C glucose 5 g/L + peptone 5 g/L + Mg SO₄, 7 H₂O 0,5 g/L + KH₂PO₄ 1 g/L + rose bengale 25 mg/L + agar 15 g/L | très légèrement ralentie | intense verte à beige |
| (4) milieu minimum à 37°C glucose 5 g/L + peptone 5 g/L + Mg SO₄, 7 H₂O 0,5 g/L + KH₂PO₄ 1 g/L + rose bengale 25 mg/L + agar 15 g/L | pas de croissance | pas de sporulation |
| (5) milieu minimum 1% glucose à 25 °C le glucose à 5 g/L du milieu minimum est augmenté à 10 g/L | ralentie | plus faible |
| (6) milieu minimum 1 % éthanol à 25 °C le glucose à 5 g/L du milieu minimum est remplacé par de l'éthanol à 10 mL/L | mycélium plus étendu mais moins dense | plus faible |
| (7) milieu minimum 1 % acide citrique à 25 °C le glucose à 5 g/L du milieu minimum est remplacé par de l'acide citrique à 10 g/L | mycélium moins dense | absente |
| (8) milieu minimum 1 % acide lactique à 25 °C le glucose à 5 g/L du milieu minimum est remplacé par de l'acide lactique à 10 g/L | légèrement ralentie | plus faible |
| (9) milieu minimum 0,2 % glycine à 25 °C la peptone à 5 g/L du milieu minimum est remplacée par de la glycine à 2 g/L | légèrement ralentie | plus faible |
| (10) milieu minimum 0,2 % oxalate d'ammonium à 25 °C la peptone à 5 g/L du milieu minimum est remplacée par de l'oxalate d'ammonium à 2 g/L | légèrement ralentie | inchangée |
| (11) milieu minimum 1 % oxalate d'ammonium à 25 °C la peptone à 5 g/L du milieu minimum est remplacée par de l'oxalate d'ammonium à 10 g/L | très ralentie | absente |
| (12) milieu minimum 0,2% urée à 25 °C la peptone à 5 g/L du milieu minimum est remplacée par de l'urée à 2 g/L | mycélium plus étendu mais moins dense | plus faible |
| (13) milieu minimum 0,2% nitrite de sodium à 25 °C la peptone à 5 g/L du milieu minimum est remplacée par du nitrite de sodium à 2 g/L | mycélium plus étendu mais moins dense | inchangée |
| (14) milieu minimum à 10 mg/l cristal violet à 25 °C | très ralentie | inchangée |
| (15) milieu minimum à 50 mg/l cristal violet à 25 °C | très ralentie | absente |
| (16) milieu malt-sulfate de cuivre à 25 °C à la lumière malt extract 10 g/L + CuSO₄, 5 H₂O 200 mg/L + agar 15 g/L | mycélium moins dense | inchangée |
| (17) milieu malt-sulfate de cuivre à 25 °C à l'obscurité malt extract 10 g/L + CuSO₄, 5 H₂O 200 mg/L + agar 15 g/L | mycélium moins dense | inchangée |
| (18) milieu Czapek-ammonium à 25 °C sucrose 30 g/L + NaNO₃ 3 g/L + K₂HPO₄ 1 g/L + MgSO₄, 7 H₂O 0,5 g/L + KCI 0,5 g/L + FeSO₄, 7 H₂O 10 mg/L + (NH₄)₂SO₄ 5 g/L + agar 15 g/L | mycélium plus étendu moins dense en zone centrale | un peu plus faible |
| (19) milieu nitrito-saccharose à 25 °C saccharose 10 g/L + NaNO₂ 2 g/L + agar 15 g/L | mycélium beaucoup moins dense | beaucoup plus faible |
| (20) milieu glycérol-nitrate à 25 °C glycérol 5 ml/L: + NaNO₃ 3g/L + agar 15 g/L | mycélium beaucoup moins dense | beaucoup plus faible |

ladite souche possédant en outre les aspects culturaux suivants :
- sur milieu PDA (Potato Dextrose Agar), il apparaît rapidement, en deux jours environ, un thalle ras translucide qui devient blanc le troisième jour avec l'apparition des conidiophores ; ce mycélium ras vire au vert très sombre au bout de cinq jours, signe d'une sporulation intense et le revers de la culture est incolore ;
- sur milieu MA (Malt Agar), son développement est légèrement différent et caractéristique de l'espèce *Trichoderma atroviride,* le mycélium forme des cercles concentriques de sporulation vert intense autour du point d'inoculation avec un thalle qui reste translucide jusqu'à la fin de la culture ; l'aspect microscopique est le même sur les deux milieux, les conidiophores sont très ramifiés avec des cellules conidiogènes (phialides) disposées en verticille par 2 ou 3, lesdites phialides sont ampulliformes, droites ou incurvées, de dimensions 2,5 à 3 µm par 8 à 14 µm et les conidies réunies au sommet de la phialide, sont globuleuses à paroi lisse, de couleur verte et mesurent environ 3 µm de diamètre ;
- ladite souche se développe entre 12°C et 36°C avec un optimum de croissance entre 20°C et 25°C sur les milieux gélosés classiques de type PDA, MA, OA (Oat Meal Agar) ;
les séquences nucléotidiques espaceurs internes transcrits 1 et 2 de ladite souche étant identiques à celles de la souche *Trichoderma atroviride* ATCC 36042.

En conséquence, un des objets de l'invention est la souche de *Trichoderma atroviride* telle que revendiquée dans la revendication 1.

Cette souche de *Trichoderma atroviride* possède les aspects culturaux suivants :
- sur milieu PDA (Potato Dextrose Agar), il apparaît rapidement, en deux jours environ, un thalle ras translucide qui devient blanc le troisième jour avec l'apparition des conidiophores ; ce mycélium ras vire au vert très sombre au bout de cinq jours, signe d'une sporulation intense et le revers de la culture est incolore ;
- sur milieu MA (Malt Agar), son développement est légèrement différent et caractéristique de l'espèce *Trichoderma atroviride*, le mycélium forme des cercles concentriques de sporulation vert intense autour du point d'inoculation avec un thalle qui reste translucide jusqu'à la fin de la culture ; l'aspect microscopique est le même sur les deux milieux, les conidiophores sont très ramifiés avec des cellules conidiogènes (phialides) disposées en verticille par 2 ou 3, lesdites phialides sont ampulliformes, droites ou incurvées, de dimensions 2,5 à 3 µm par 8 à 14 µm et les conidies réunies au sommet de la phialide, sont globuleuses à paroi lisse, de couleur verte et mesurent environ 3 µm de diamètre ;
- ladite souche se développe entre 12 °C et 36 °C avec un optimum de croissance entre 20 °C et 25 °C sur les milieux gélosés classiques de type PDA, MA, OA (Oat Meal Agar).

Un objet de l'invention est également un milieu de culture caractérisé en ce qu'il comprend au moins un *Trichoderma atroviride* selon la revendication 1 et au moins un substrat, particulièrement un substrat d'origine végétale et plus particulièrement un substrat riche en polyholosides tels que l'amidon, l'hémicellulose ou la cellulose.

De préférence, ce milieu de culture comprend au moins un diluant minéral inerte.

De manière préférée, ce diluant est spécifiquement sélectionné pour diluer et/ou aérer ledit milieu de culture, par exemple, ledit diluant est choisi dans le groupe constitué par les argiles calcinées en grains telles que l'attapulgite, les billes d'argile soufflées, les poudres de roches volcaniques comme la pouzzolane ou la pierre ponce, la perlite expansée, la vermiculite expansée et la laine de roche.

Préférentiellement, le milieu de culture selon l'invention comprend au moins un élément minéral utile à la croissance du *Trichoderma atroviride* tel qu'un nitrate, de l'ammonium, un orthophosphate, du potassium, du magnésium, du calcium ou un oligo-élément.

Selon un mode de réalisation préféré, ce milieu de culture comprend un acide, de préférence minéral, permettant de maintenir le pH du milieu entre 2 et 4 et de préférence entre 2,7 et 3,3.

Un autre objet de l'invention est un concentré déshydraté d'un *Trichoderma atroviride* selon la revendication 1, particulièrement sous forme de poudre.

L'invention a également pour objet un procédé d'obtention d'une poudre à base du *Trichoderma atroviride* selon la revendication 1, ledit procédé comprenant les étapes de fermentation du milieu de culture selon l'invention, de déshydratation à basse température, suivies d'une étape de broyage.

Un autre objet de l'invention concerne l'utilisation du *Trichoderma atroviride* selon la revendication 1 comme stimulant de la germination et/ou de la croissance des plantes.

L'invention a également pour objet l'utilisation du *Trichoderma atroviride* selon la revendication 1en tant qu'agent de biocontrôle, notamment contre les champignons pathogènes des plantes.

Il convient de noter que, concernant les deux utilisations précitées, le *Trichoderma atroviride* selon la revendication 1, pourra être administré, entre autres, directement sous une forme appropriée et plus particulièrement sous forme de poudre. Cette poudre pourra être du champignon brut mais préférablement sera la poudre obtenue via le procédé d'obtention précité, c'est-à-dire après fermentation du milieu de culture précité, déshydratation à basse température, puis broyage. Un autre objet de l'invention consiste en un procédé d'isolement du *Trichoderma atroviride* selon la revendication 1, caractérisé en ce que ledit procédé comprend les étapes successives suivantes :
a) Prélèvement d'un extrait de champignon *Trichoderma atroviride* dans un milieu adéquat comme un sol inculte ou cultivé, sur les plantes ou dans les bois en décomposition issus de ceps de vigne, de branches ou de troncs d'arbres,
b) Dilution de l'extrait prélevé avec de l'eau stérile,
c) Culture de cet extrait sur au moins un milieu sélectif, ledit milieu sélectif étant compris dans le groupe constitué par : le milieu MA2 (malt agar 2%) et le milieu RB-S-F (McFadden et Sutton),
d) Repiquage des champignons *Trichoderma atroviride* issus de spores isolées dans des boîtes de Pétri contenant du milieu PDA (Potato Dextrose Agar),
e) Culture des champignons *Trichoderma atroviride* repiqués durant douze jours dans les conditions de culture suivantes :
   - (1) milieu PDA à 25 °C, à 39 g/L;
   - (2) milieu minimum à 4 °C, comprenant du glucose à 5 g/L, de la peptone à 5 g/L, du Mg SO₄, 7 H₂O à 0,5 g/L, du KH₂PO₄ à 1 g/L, du rose bengale à 25 mg/L, de l'agar à 15 g/L;
   - (3) milieu minimum à 25 °C, de composition identique à celle du milieu minimum (2);
   - (4) milieu minimum à 37 °C, de composition identique à celle du milieu minimum (2);
   - (5) milieu minimum 1 % glucose à 25 °C, le glucose à 5 g/L du milieu minimum étant augmenté à 10 g/L;
   - (6) milieu minimum 1 % éthanol à 25 °C, le glucose à 5 g/L du milieu minimum étant remplacé par de l'éthanol à 10 mL/L;
   - (7) milieu minimum 1 % acide citrique à 25 °C, le glucose à 5 g/L du milieu minimum étant remplacé par de l'acide citrique à 10 g/L;
   - (8) milieu minimum 1 % acide lactique à 25 °C, le glucose à 5 g/L du milieu minimum étant remplacé par de l'acide lactique à 10 g/L;
   - (9) milieu minimum 0,2 % glycine à 25 °C, la peptone à 5 g/L du milieu minimum étant remplacée par de la glycine à 2 g/L;
   - (10) milieu minimum 0,2 % oxalate d'ammonium à 25 °C, la peptone à 5 g/L du milieu minimum étant remplacée par de l'oxalate d'ammonium à 2 g/L;
   - (11) milieu minimum 1 % oxalate d'ammonium à 25 °C, la peptone à 5 g/L du milieu minimum étant remplacée par de l'oxalate d'ammonium à 10 g/L;
   - (12) milieu minimum 0,2 % d'urée à 25 °C, la peptone à 5 g/L du milieu minimum étant remplacée par de l'urée à 2 g/L;
   - (13) milieu minimum 0,2 % nitrite de sodium à 25 °C, la peptone à 5 g/L du milieu minimum étant remplacée par du nitrite de sodium à 2 g/L;
   - (14) milieu minimum avec cristal violet à 10 mg/L à 25 °C;
   - (15) milieu minimum avec cristal violet à 50 mg/L à 25 °C;
   - (16) milieu malt-sulfate de cuivre à 25 °C à la lumière, comprenant du malt extract à 10 g/L, du CuSO₄, 5 H₂O à 200 mg/L, de l'agar à 15 g/L)
   - (17) milieu malt-sulfate de cuivre à 25 °C à l'obscurité, de composition identique à celle du milieu (16);
   - (18) milieu Czapek-ammonium à 25 °C, comprenant du sucrose à 30 g/L, du NaNO₃ à 3 g/L, du K₂HPO₄ à 1 g/L, du MgSO₄, 7 H₂O à 0,5 g/L, du KCl à 0,5 g/L, du FeSO₄, 7 H₂O à 10 mg/L, du (NH₄)₂SO₄ à 5 g/L, de l'agar à 15 g/L);
   - (19) milieu nitrito-saccharose à 25 °C, comprenant du saccharose à 10 g/L, du NaNO₂ à 2 g/L, de l'agar à 15 g/L;
   - (20) milieu glycérol-nitrate à 25 °C, comprenant du glycérol à 5 ml/L, du NaNO₃ à 3g/L, de l'agar à 15 g/L;
      Afin de déterminer leur profil biochimique, morphologique et physiologique;
f) Comparer le profil biochimique, morphologique et physiologique desdits champignons avec le profil biochimique, morphologique et physiologique spécifique de la souche *Trichoderma atroviride* selon la revendication 1; et
g) Isoler les champignons *Trichoderma atroviride* dont le profil biochimique, morphologique et physiologique correspond au profil biochimique, morphologique et physiologique spécifique de la souche *Trichoderma atroviride* selon la revendication 1.

La description détaillée ci-après a pour but d'exposer l'invention de manière suffisamment claire et complète, notamment à l'aide d'exemples, mais ne doit pas être considérée comme limitant l'étendue de la protection aux modes de réalisation particuliers et aux exemples présentés.

### Exemple d'extraction de Trichoderma atroviride MUCL 45632

Selon un mode préférentiel l'extraction de *Trichoderma atroviride* MUCL 45632 est réalisée partir du sol.

Les prélèvements sont effectués jusqu'à 30 à 40 cm de profondeur, correspondant à la rhizosphère, à l'aide d'ustensiles préalablement désinfectés à l'alcool. La terre ainsi récoltée est tamisée sur tamis de 2 mm afin d'éliminer les gros débris (cailloux, débris racinaires et feuilles). Les échantillons de terre sont séchés à 25 °C pendant 3 jours. Ils sont ensuite utilisés immédiatement pour la recherche de champignon du genre *Trichoderma*.

L'extraction est réalisée par la technique des « suspensions-dilutions ». Cela consiste à mettre 10 g de chaque échantillon dans 90 ml d'eau distillée stérile, puis à agiter pendant 30 minutes. Cet extrait correspond à la suspension mère. Les dilutions sérielles de suspension mère peuvent aller jusqu'à la dilution 10⁻⁶. Il n'est pas utile d'aller plus loin dans les dilutions car les sols en friche ne sont pas extrêmement riches en *Trichoderma.*

Dans cet exemple la culture est réalisée sur deux types de milieux sélectifs :

| *Milieu MA2 (malt agar 2%)* | |
|---|---|
| extrait de malt | 20 g |
| Agar | 20 g |
| Eau | 1000 mL |
| | |
| *Milieu RB-S-F (Mc Fadden et Sutton)* | |
| KH₂PO₄ | 1 g |
| MgSO₄,7H₂O | 0,5 g |
| Peptone | 5 g |
| Glucose | 10 g |
| rose bengale | 17 mg |
| sulfate de streptomycine | 30 mg |
| Gélose | 20 g |
| Eau | 1000 mL |
| Formol ajouté après autoclavage | 0,2 mL |

Deux séries de boîtes de Pétri reçoivent respectivement des volumes de 1 mL des dilutions 10⁻⁴, 10⁻⁵ et 10⁻⁶ de chaque échantillon. Les milieux sélectifs sont coulés en surfusion à 45 °C sur les dépôts de liquide dans chaque série de boites de Pétri. L'homogénéisation est faite manuellement par un mouvement circulaire des boîtes. Les boîtes sont laissées sous la hotte à flux laminaire jusqu'à leur complet durcissement. Elles sont ensuite placées dans une étuve à 25 °C et observées quotidiennement jusqu'à l'apparition des mycéliums sans confluence.

### Isolement monosporal et identification

A partir des mycéliums non confluents apparus sur les deux milieux de sélection, on repique uniquement les champignons issus de spores isolées dans des boîtes de Pétri contenant du milieu PDA (Potato Dextrose Agar) puis on les cultive dans une étuve à 25 °C.

| *PDA (Potato Dextrose Agar)* | |
|---|---|
| extrait de pommes de terre | 1000 mL |
| glucose | 20 g |
| agar | 20 g |

Après 3 à 4 jours de culture, quand les hyphes ont commencé à couvrir la surface de la boîte, on procède à leur identification par observation microscopique par la technique du ruban adhésif :
Sous une hotte à flux laminaire, la face collante d'un petit morceau de ruban adhésif transparent est appliquée légèrement sur le mycélium afin d'en récupérer quelques fragments. Le ruban adhésif est déposé sur une lame porte-objet sur laquelle on a préalablement mis une goutte de colorant bleu coton au lactophénol. On met une autre goutte de colorant sur le ruban adhésif puis on place une lamelle dessus. L'observation du montage est effectuée sous microscope aux grossissements 100 ou 400.

| *Colorant bleu coton au lactophénol* |
|---|
| 50 mg de bleu coton (ou bleu de méthyle, acid blue 93, C.I. n° 42780) |
| dissous dans 100 ml de lactophénol d'Amann (25 g de phénol pur cristallisé dissous à chaud dans 25 ml d'eau distillée, 25 g d'acide lactique 85 % et 50 g de glycérol anhydre) |

Grâce à cette méthode, la souche *Trichoderma atroviride* MUCL 45632 a été identifiée. Il est apparu que cette souche possédait des propriétés particulièrement avantageuses notamment en matière de compétition avec les pathogènes, de viabilité, de résistance aux toxiques, etc.

### Identification génétique de Trichoderma atroviride MUCL 45632

La technique employée pour l'identification génétique de la souche *Trichoderma atroviride* MUCL 45632 a été le séquençage des espaceurs internes transcrits 1 et 2 (ITS 1 et 2). Cette technique, largement répandue et fréquemment utilisée, permet de caractériser certaines espèces animales ou végétales en fonction de la variabilité génétique de ces séquences. En l'espèce, le séquençage des ITS 1 et 2 de cette nouvelle souche a permis de confirmer qu'elle appartenait à l'espèce *Trichoderma atroviride* en raison de l'identité de ses séquences nucléotidiques ITS avec celles d'autres *Trichoderma atroviride* (telle la souche ATCC 36042, n° d'accession (Genbank) de la séquence des ITSs : AF278796).

### Identification biochimique de la souche Trichoderma atroviride MUCL 45632 et comparaison avec d'autres Trichoderma

### Identification biochimique

Les souches de *Trichoderma* peuvent être identifiées et comparées en les cultivant sur différents milieux et dans différentes conditions. L'analyse se fait selon deux critères : l'aspect des mycéliums et l'effet du milieu de culture (inhibition ou stimulation de la croissance et/ou de la sporulation de la souche). Les cultures durent 12 jours. Les conditions de culture utilisées pour la comparaison sont :
(1)milieu PDA à 25 °C (potato dextrose agar Sigma 39 g/L)
(2)milieu minimum à 4 °C, (glucose 5 g/L + peptone 5 g/L + Mg SO₄, 7 H₂O 0,5 g/L + KH₂PO₄ 1 g/L + rose bengale 25 mg/L + agar 15 g/L)
(3)milieu minimum à 25 °C (idem)
(4)milieu minimum à 37 °C (idem)
(5)milieu minimum 1 % glucose à 25 °C (le glucose à 5 g/L du milieu minimum est augmenté à 10 g/L)
(6)milieu minimum 1 % éthanol à 25 °C (le glucose à 5 g/L du milieu minimum est remplacé par de l'éthanol à 10 mL/L)
(7)milieu minimum 1 % acide citrique à 25 °C (le glucose à 5 g/L du milieu minimum est remplacé par de l'acide citrique à 10 g/L)
(8)milieu minimum 1 % acide lactique à 25 °C (le glucose à 5 g/L du milieu minimum est remplacé par de l'acide lactique à 10 g/L)
(9)milieu minimum 0,2 % glycine à 25 °C (la peptone à 5 g/L du milieu minimum est remplacée par de la glycine à 2 g/L)
(10)milieu minimum 0,2 % oxalate d'ammonium à 25 °C (la peptone à 5 g/L du milieu minimum est remplacée par de l'oxalate d'ammonium à 2 g/L)
(11)milieu minimum 1 % oxalate d'ammonium à 25 °C (la peptone à 5 g/L du milieu minimum est remplacée par de l'oxalate d'ammonium à 10 g/L)
(12)milieu minimum 0,2 % d'urée à 25 °C (la peptone à 5 g/L du milieu minimum est remplacée par de l'urée à 2 g/L)
(13)milieu minimum 0,2 % nitrite de sodium à 25 °C (la peptone à 5 g/L du milieu minimum est remplacée par du nitrite de sodium à 2 g/L)
(14)milieu minimum avec cristal violet à 10 mg/L à 25 °C
(15)milieu minimum avec cristal violet à 50 mg/L à 25 °C
(16)milieu malt-sulfate de cuivre à 25 °C à la lumière (malt extract 10 g/L + CuSO₄, 5 H₂O 200 mg/L + agar 15 g/L)
(17)milieu malt-sulfate de cuivre à 25 °C à l'obscurité (idem)
(18)milieu Czapek-ammonium à 25 °C (sucrose 30 g/L + NaNO₃ 3 g/L + K₂HPO₄ 1 g/L + MgSO₄, 7 H₂O 0,5 g/L + KCI 0,5 g/L + FeSO₄, 7 H₂O 10 mg/L + (NH₄)₂SO₄ 5 g/L + agar 15 g/L)
(19)milieu nitrito-saccharose à 25 °C (saccharose 10 g/L + NaNO₂ 2 g/L + agar 15 g/L)
(20)milieu glycérol-nitrate à 25 °C (glycérol 5 ml/ : + NaNO₃ 3g/L + agar 15 g/L)

Le profil biochimique de la souche *Trichoderma atroviride* MUCL 45632 est résumé dans le tableau qui suit.

| Milieu de culture | Vitesse de croissance | Aspect de la sporulation |
|---|---|---|
| (1)milieu de référence PDA 25°C | très rapide | vert intense |

| Milieu de culture | Vitesse de croissance par rapport au milieu PDA | Aspect de la sporulation par rapport au milieu PDA |
|---|---|---|
| (2) milieu minimum 4°C | très lente | absente |
| (3) milieu minimum 25°C | très légèrement ralentie | intense verte à beige |
| (4) milieu minimum 37°C | pas de croissance | pas de sporulation |
| (5) milieu minimum 1% glucose 25 °C | ralentie | plus faible |
| (6) milieu minimum 1 % éthanol 25 °C | mycélium plus étendu mais moins dense | plus faible |
| (7) milieu minimum 1 % acide citrique 25 °C | mycélium moins dense | absente |
| (8) milieu minimum 1 % acide lactique 25 °C | légèrement ralentie | plus faible |
| (9) milieu minimum 0,2 % glycine 25 °C | légèrement ralentie | plus faible |
| (10) milieu minimum 0,2 % oxalate d'ammonium 25 °C | légèrement ralentie | inchangée |
| (11) milieu minimum 1% oxalate d'ammonium 25 °C | très ralentie | absente |
| (12) milieu minimum 0,2% urée 25 °C | mycélium plus étendu mais moins dense | plus faible |
| (13) milieu minimum 0,2% nitrite de sodium 25 °C | mycélium plus étendu mais moins dense | inchangée |
| (14) milieu minimum à 10 mg/l cristal violet 25 °C | très ralentie | inchangée |
| (15) milieu minimum à 50 mg/l cristal violet 25 °C | très ralentie | absente |
| (16) milieu extrait de maltsulfate de cuivre à la lumière 25 °C | mycélium moins dense | inchangée |
| (17) milieu extrait de malt-sulfate de cuivre à l'obscurité 25 °C | mycélium moins dense | inchangée |
| (18) milieu Czapek-ammonium 25 °C | mycélium plus étendu moins dense en zone centrale | un peu plus faible |
| (19) milieu nitrito-saccharose 25 °C | mycélium beaucoup moins dense | beaucoup plus faible |
| (20) milieu glycérol-nitrate 25 °C | mycélium beaucoup moins dense | beaucoup plus faible |

Le profil est unique. Il est influencé par le genre, l'espèce et la variété du champignon. Il permet d'identifier spécifiquement chaque souche de champignon *Trichoderma.*

### Comparaison avec diverses souches de Trichoderma :

Dans cet exemple, les souches de *Trichoderma* étudiées sont :
- MUCL 45632 (*T*. *atroviride*, identifié par séquençage des ITS-1 et caractérisé à l'aide de tests biochimiques ; souche isolée par la demanderesse d'un sol en friche)
- CNCM I-1571 (*T*. *atroviride*, identifiée par séquençage des ITS-1, Brevet FR2780972)
- T-39 (CNCM I-952) (*Trichoderma harzianum* Rifai, identifiée par séquençage des ITS-1, Brevet EP0466133)
- T-22 (1295-22, ATCC 20847) (*Trichoderma harzianum* Rifai, identifiée par séquençage des ITS-1, Brevet EP0285987)
- TV1 (Nixe 13602A) (*T*. *harzianum*, identifiée par séquençage des ITS-1, souche de produit commercial Agribiotec, Italie)
- Nixe 00401A (*T*. *atroviride,* identifiée par séquençage des ITS-1, souche isolée par la demanderesse d'une culture de champignons *Pleurotus ostreatus)*
- Nixe 30301E (*Trichoderma* sp., souche isolée par la demanderesse d'une culture hors-sol *d'Allium porum*)

### Contrôle des similitudes d'aspect des colonies sur boîtes de Pétri :

Les boites obtenues avec chaque condition de culture sont comparées deux à deux. Pour chaque condition de culture, on attribue la note 1 lorsque deux souches donnent des colonies d'aspect similaire pour une condition de culture donnée.

Lorsque l'aspect est différent, la note est 0. Il y a 20 tests de culture au total. Pour chaque couple, l'ensemble des notes est additionné. La note maximale est 20.

Exemples : note 7/20 - la souche I-1571 est trouvée 7 fois associée avec la souche MUCL 45632, c'est-à-dire qu'elles donnent des colonies d'aspect similaire dans sept conditions de culture différentes ; note 0/20 - la souche T-39 n'est jamais associée à la souche MUCL 45632.

**Tableau des notations comparées des similitudes d'aspect (sur 20) :**

| **MUCL 45632** | | | | | | |
|---|---|---|---|---|---|---|
| *7* | **I-1571** | | | | | |
| *0* | *0* | **T-39** | | | | |
| *0* | *0* | *6* | **T-22** | | | |
| *1* | *1* | ***18*** | *4* | **TV1** | | |
| *5* | ***15*** | *1* | *0* | *2* | **00401A** | |
| *0* | *2* | *5* | ***11*** | *5* | *2* | **30301E** |

La souche MUCL 45632 est apparentée aux souches I-1571 (7/20) et 00401A (5/20). Les souches I-1571 et 00401A sont proches (15/20). Ces 3 souches sont des *T. atroviride*, génétiquement proches comme cela a été confirmé par séquençage des ITS-1 (INRA Versailles), mais sont toutefois différentes et différenciables.

Les souches *T*. *harzianum* T-39 et TV1 sont très proches l'une de l'autre (18/20) suggérant une origine commune. La souche 30301E, isolée d'une culture *d'Allium porum,* non séquencée, est proche de la souche T-22 et donc d'un *Trichoderma harzianum.* Les souches T-39, TV1, T-22 et 30301E forment un autre groupe de type *T. harzianum* au sein duquel les souches T-22 et T-39 se distinguent bien entre elles.

### Contrôle de l'effet des milieux sur les souches :

Pour chaque condition de culture, les souches ont été comparées pour les effets de stimulation ou d'inhibition par les milieux de culture et d'intensité de sporulation. Les variations de croissance et de sporulation sont estimées par rapport à la référence : milieu PDA à 25°C. Il y a 20 tests de culture au total. Pour chaque condition de culture, on attribue la note 1 à un couple lorsque le comportement est identique et 0 lorsque le comportement est différent.

Le tableau suivant récapitule toutes les associations réalisées. Comme précédemment, la note totale correspond à la somme des réponses pour chaque couple. La note maximale est 20.

**Tableau des notations comparées de l'effet des milieux (sur 20) :**

| **MUCL 45632** | | | | | | |
|---|---|---|---|---|---|---|
| ***12*** | **I-1571** | | | | | |
| *7* | *9* | **T-39** | | | | |
| *7* | *7* | ***13*** | **T-22** | | | |
| *7* | *8* | *13* | *9* | **TV1** | | |
| ***11*** | ***13*** | *9* | ***8*** | ***11*** | **00401A** | |
| *6* | *8* | *9* | *8* | *8* | *10* | **30301E** |

Les notes sont moyennes et proches les unes des autres. Ce test montre que les souches sont toutes des *Trichoderma* sp., mais différentes les unes des autres. Les souches MUCL 45632, I-1571 et 00401A forment un groupe hétérogène et les souches T-39, T-22 et TV1 un autre groupe hétérogène. Les souches TV1 et 00401A peuvent être rapprochées.

### Moyenne géométrique des notations des tests :

La moyenne géométrique des notes de chacun des couples permet de distinguer clairement les souches entre elles. La moyenne géométrique est la racine carrée du produit des deux notes obtenues précédemment.

**Tableau des moyennes géométriques des notations comparées des aspects de colonies et des effets des milieux :**

| **MUCL 45632** | | | | | | |
|---|---|---|---|---|---|---|
| *9* | **I-1571** | | | | | |
| *0* | *0* | **T-39** | | | | |
| *0* | *0* | *9* | **T-22** | | | |
| *3* | *3* | ***15*** | *6* | **TV1** | | |
| *7* | **14** | *3* | *0* | *5* | **00401A** | |
| *0* | *4* | *7* | *9* | *6* | *4* | **30301E** |

- Les résultats montrent un regroupement des souches par espèce, mais il n'y a jamais identité complète au sein d'un couple.
- Les souches I-1571 et 00401A sont assez proche. Il en est de même pour les souches T-39 et TV1.
- La souche *T. atroviride* MUCL 45632 se distingue clairement de toutes les autres et plus particulièrement des souches *T. harzianum* Rifai.

### Conditions de culture du champignon Trichoderma atroviride MUCL 45632

### Choix du mode de culture

Dans le milieu naturel, les champignons sont des micro-organismes qui se développent sur des substrats solides et humides. Pour la production industrielle, il existe deux options : la culture submergée en milieu liquide ou aérienne sur milieu solide. Le choix peut être fait notamment à partir de nos essais en laboratoire et des travaux de G.A. Munoz. et al. (FEMS Microbiology Letters, 125, 63-70, 1995) et de A. Durand. (Les colloques de l'INRA, 18, 263-277, 1983 et Biofutur, 181, 41-43, 1998).

La culture doit donner un produit concentré qui conserve bien son activité. Les formes de conservation de *Trichoderma atroviride* sont les conidiospores et les chlamydospores. Le mycélium ne survit pas longtemps à la dessiccation et au manque de nutriments.

Les essais de culture submergée en milieu liquide en fermenteur agité, en secoueur ou cytoculteur ne permettent pas une bonne production de spores dans des milieux spécifiques des champignons. Le champignon *T. atroviride* se développe en surface et il sporule mal. La culture en milieu liquide donne essentiellement du mycélium et des chlamydospores. Les spores de *T. atroviride* obtenues en milieu liquide ont des parois fines et incomplètes. Elles sont moins résistantes aux stress malgré leur richesse en tréhalose. Leur pouvoir germinatif diminue à moins de 15 % après 45 jours de stockage sous une humidité relative (HR) de 75 % (a_{w} 0,75). Elles sont hydrophiles et peu adhérentes. Elles sont peu résistantes aux fortes doses d'UV. Les spores cultivées en milieu liquide interagissent plus facilement avec le milieu (eau et nutriments) ce qui permet une réponse rapide avec un temps de germination plus court que pour les spores aériennes. Mais cela les rend aussi beaucoup plus sensibles aux détériorations.

La culture submergée en milieu liquide n'est pas bien adaptée à la production du champignon *Trichoderma.*

La culture sur milieu solide reproduit les conditions naturelles. Elle est adaptée aux champignons filamenteux notamment *Trichoderma*. Pour se développer, les champignons ont besoin d'air et d'une humidité sans excès, contrairement aux bactéries et les meilleures conditions sont de l'air humide contenant 61 à 94 % d'humidité relative (a_{w} de 0,61 à 0,94). Ces conditions, peu favorables aux contaminants bactériens qui ont besoin d'une humidité élevée (a_{w} 0,91 à 1,0), permettent de travailler avec des mesures de stérilité simplifiées.

Les études structurelles et biochimiques montrent que les spores de *T. atroviride* sont de meilleure qualité en culture en milieu solide. Elles offrent une plus grande résistance aux stress et leur viabilité est pleinement maintenue pendant 45 jours de stockage sous une humidité relative de 75 % (a_{w} 0,75). De plus, elles sont capables de résister à de fortes doses d'UV, ce qui semble être en relation avec l'épaisseur de leur paroi et avec les pigments vert foncé qui se trouvent à la surface des spores. Le taux de tréhalose des spores aériennes est plus faible que celui des spores submergées (0,5 % contre 5,6 % selon G.A. Munoz, 1995, *supra*). Bien que le tréhalose soit connu comme agent stabilisant des spores, il ne semble pas jouer sur la qualité des spores submergées et l'élévation du taux de tréhalose est plus la caractéristique d'un stress lors de la culture qu'un avantage particulier de conservation. Les spores aériennes présentent un état dormant naturel qui les rend résistantes.

Les essais montrent que les niveaux de production par unité de volume peuvent être plus importants en milieu solide qu'en culture submergée.

Le principal inconvénient que présente la culture en milieu solide sur de gros volumes est la régulation de la température car au cours de leur croissance, les micro-organismes produisent de la chaleur métabolique qui ne peut pas être évacuée par circulation du milieu de culture. Cependant, l'insertion de surfaces d'échange de chaleur dans le lit de culture et la ventilation de la masse de substrat permettent le contrôle de la température et évitent la mort du champignon par surchauffe.

La production de spores en milieu solide donne de meilleurs produits avec un meilleur rendement mais demande un équipement de manutention plus complexe qu'en milieu liquide. Le rendement et la qualité des spores sont des critères essentiels dans la production de *T. atroviride* MUCL 45632 et ainsi la culture en milieu solide est préférée à celle en milieu liquide, malgré les problèmes liés à la manipulation des solides.

### Mode opératoire pour la production du Trichoderma en fermenteur en milieu solide

Le milieu de culture est essentiellement constitué d'un substrat d'origine végétale riche en polyholosides tels l'amidon, l'hémicellulose et la cellulose. Ce substrat pourra être constitué de manière non limitative, de graines de céréales (orge, avoine) ou de légumineuses (soja, lupin, fèves), de sous-produits de l'industrie des céréales (son de céréales, germes de blé, coques de riz), de tourteaux d'extraction d'oléagineux (tournesol, coton), de déchets de l'industrie du sucre et de l'industrie de l'amidon (pulpe de betterave, bagasse de canne à sucre), de paille ou de copeaux de bois. Le substrat végétal doit être broyé grossièrement pour former une masse perméable à l'eau et à l'air.

Aux substances végétales, on pourra ajouter des diluants minéraux inertes destinés à diluer et à aérer le milieu. Ces diluants pourront être choisis de manière non limitative parmi les argiles calcinées en grains (attapulgite), les billes d'argile soufflées, les poudres de roches volcaniques (pouzzolane, pierre ponce), la perlite expansée, la vermiculite expansée, la laine de roche.

Le milieu pourra être complémenté en éléments minéraux utiles à la croissance du champignon : nitrate, ammonium, ortho-phosphate, potassium, magnésium, calcium et oligo-éléments.

Le pH sera avantageusement acidifié à l'aide d'un acide de préférence minéral, comme l'acide chlorhydrique, l'acide sulfurique ou l'acide ortho-phosphorique, entre pH 2,7 et pH 4 et de préférence entre 2,7 et 3,3.

Un milieu de culture particulièrement approprié est composé de :

| *Milieu Pulpe de betterave* | | |
|---|---|---|
| Pulpe de betterave déshydratée, | 25,7 | kg |
| Perlite expansée | 25,7 | kg |
| Acide sulfurique 92 % | 1,2 | kg |
| Eau | 47,4 | L |

Ce milieu est soigneusement mélangé puis il est désinfecté par chauffage à 100 °C pendant 2 heures directement dans le fermenteur. Le pH final après refroidissement est voisin de 3. Le milieu est ensemencé à froid à l'aide d'une suspension de spores de *T*. *atroviride* dans l'eau stérile titrant entre 10⁶ et 10⁷ spores/mL. Le volume de suspension utilisé est 10 L pour 100 kg de milieu désinfecté. La fermentation dure de 2 à 3 semaines. Le fermenteur est alimenté en air stérile avec un débit constant de 12 L/mn pour 100 kg de milieu. La température du milieu est maintenue entre 12 et 35°C, de préférence en dessous de 34 °C, de manière particulièrement préférée de 20 à 25°C. En fin de culture la concentration en spores viables atteint 1 à 5.10⁹ spores viables/g. Le substrat fermenté est ensuite déshydraté à basse température avant d'être broyé pour obtenir une poudre. La taille des grains qui compose cette poudre est variable. On pourra préparer une poudre fine (granulométrie inférieure à 100 µm) ou une poudre plus grossière (diamètre moyen de1 à 3 mm), en fonction des usages.

Cette poudre pourra être ensuite dispersée, préalablement à son administration, dans un liquide tel que de l'eau puis sera appliquée par pulvérisation ou aspersion sur les parties aériennes et/ou les racines des plantes.

Alternativement, cette poudre peut être incorporée directement dans le support de culture des plantes avant ou après plantation.

Alternativement, cette poudre peut être mélangée à du terreau ou tout autre support de culture, le mélange sera alors déposé au pied de la plante.

Selon un mode de réalisation particulièrement préféré, la poudre est filtrée et/ou tamisée afin d'enlever les grosses particules. Ensuite, la poudre ainsi tamisée peut être incorporée dans de la terre ou du terreau, comme susmentionné, ou être appliquée directement sur le feuillage des plantes à traiter. Bien évidemment, cette poudre fine pourrait également être dispersée et appliquée sur les plantes par aspersion.

Les poudres précitées pourront avoir différentes utilisations. Elles pourront notamment être appliquées aux plantes à des fins phytosanitaires. Une telle application peut trouver un intérêt notamment dans la lutte biologique et notamment la lutte contre certains champignons pathogènes.

Selon un mode de réalisation particulièrement préféré, ces poudres pourront être utilisées pour stimuler la germination et/ou la croissance des graines et/ou des plantes. En effet, d'excellents résultats ont été obtenus pour de telles utilisations.

### Etude de la stabilité de conservation des spores de MUCL 45632 à température ambiante

La stabilité de la viabilité est une propriété remarquable de la souche *Trichoderma atroviride* MUCL 45632.

Dans les conditions habituelles de fabrication, le champignon MUCL 45632 est multiplié sur un substrat de culture solide. A l'issue de la période de fermentation, le substrat chargé en spores est séché à basse température. Le produit fini déshydraté contient environ 8,5 % (poids) de spores de champignon. La viabilité des spores de la souche MUCL 45632 dans le produit fini a été étudiée sur plusieurs lots de production d'essai pendant plusieurs mois. Les spores viables sont dénombrées selon la norme NF ISO 7954. Les résultats ont été obtenus sur 5 lots successifs dans les mêmes conditions de production.

Les lots ont été stockés à température ambiante (de 18 à 28 °C), à la lumière naturelle et dans des sacs transparents scellés.

### Spores viables par gramme de produit sec :

| temps de stockage | | | | | |
|---|---|---|---|---|---|
| lots (date production) | 0 mois | 1 mois | 2 mois | 3 mois | 4 mois |
| **B26 (été 2005)** | *3,0.10⁹* | *2,8.10⁹* | *1,6.10⁹* | *1,0.10⁹* | *2,3.10⁹* |
| **B27 (été 2005)** | *2,0.10⁹* | *1,5.10⁹* | *1,6.10⁹* | *2,6.10⁹* | *1,0.10⁹* |
| **B28 (automne 2005)** | *1,4.10⁹* | *2,5.10⁹* | *1,9.10⁹* | *2,6.10⁹* | *2,1.10⁹* |
| **B29 (automne 2005)** | *2,0.10⁹* | *1,5.10⁹* | *1,9.10⁹* | *2,3.10⁹* | *3,0.10⁹* |
| **B30 (automne 2005)** | *3,2.10⁹* | *2,6.10⁹* | *2,2.10⁹* | *1,6.10⁹* | *1,0*.*10⁹* |
| moyenne | *2,3.10⁹* | *2,2.10⁹* | *1,8.10⁹* | *2,0.10⁹* | *1,9.10⁹* |
| Ecart-type | *0,7.10⁹* | *0,6.10⁹* | *0,3.10⁹* | *0,6.10⁹* | *0,8.10⁹* |

Les lots conservent tous une viabilité supérieure ou égale à 10⁹ spores/g après 4 mois. En moyenne, la concentration en spores viables ne change pas au cours des 4 mois d'observation.

Le produit fini à base de *T. atroviride* MUCL 45632 conserve toute sa viabilité pendant au minimum 4 mois sans artifice, contrairement à des produits similaires qui réclamant un stockage au réfrigérateur ou au congélateur ou bien l'ajout de régulateurs osmotiques.

La stabilité de la viabilité de la souche MUCL 45632 est une propriété particulièrement remarquable qui répond aux attentes des utilisateurs qui recherchent des produits que l'on peut transporter et stocker sans risque de perte à température ambiante et sans le problème de respect de la chaîne du froid.

### Effet de la température sur la croissance de la souche MUCL 45632

Les champignons *Trichoderma* sont utilisés dans la rhizosphère ou sur le feuillage des plantes. Ils sont soumis à des températures variables. Il est important de connaître la plage de température de croissance et il est préférable que celle-ci soit la plus large possible.

L'étude de l'effet de la température consiste à mettre des bouchons mycéliens de *Trichoderma* sur des boîtes de Pétri gélosées au PDA. Les boîtes sont ensuite déposées dans des incubateurs à 4, 14, 20, 25, 30 et 37 °C. La culture dure 15 jours. Ensuite, elles sont observées pour comparer leur état de développement et de sporulation.

Dans cet exemple, les souches de *Trichoderma* étudiées sont :
- MUCL 45632 (*T. atroviride*, identifiée ITS-1, souche isolée par la demanderesse d'un sol en friche)
- T-39 (CNCM I-952) (*Trichoderma harzianum* Rifai, identifiée ITS-1, Brevet EP0466133)
- T-22 (1295-22, ATCC 20847) (*T. harzianum* Rifai, identifiée ITS-1, Brevet EP0285987)
- Nixe 30301E (*Trichoderma* sp., souche isolée par la demanderesse d'une culture hors-sol d'*Allium porum*)
- Inra AP (*Trichoderma* sp., collection Mme Dubos, INRA, UMR Santé Végétale, Villenave d'Ornon, France)
- Inra APS (*Trichoderma* sp., collection Mme Dubos, INRA, UMR Santé Végétale, Villenave d'Ornon, France)
- Inra B11 (*Trichoderma* sp., collection Mme Dubos, INRA, UMR Santé Végétale, Villenave d'Ornon, France)
- Inra P1 (*Trichoderma* sp., collection Mme Dubos, INRA, UMR Santé Végétale, Villenave d'Ornon, France)

*Notation de la croissance et de la sporulation à différentes températures :*

| | température d'incubation | | | | | |
|---|---|---|---|---|---|---|
| Souche | 4 °C | 14 °C | 20 °C | 25 °C | 30 °C | 37 °C |
| **MUCL 45632** | *0* | *2* | *2* | *2* | *2* | *0* |
| **T-39** | *0* | *1* | *1* | *2* | *2* | *0* |
| **T-22** | *0* | *1* | *1* | *2* | *2* | *0* |
| **30301E** | *0* | *1* | *1* | *2* | *2* | *2* |
| **AP** | *0* | *1* | *2* | *2* | *2* | *0* |
| **APS** | *0* | *1* | *2* | *2* | *2* | *0* |
| **B11** | *0* | *1* | *2* | *2* | *2* | *0* |
| **P1** | *0* | *1* | *2* | *2* | *2* | *0* |

| | | | | | | |
|---|---|---|---|---|---|---|
| *Notation : 0 pas de* croissance *; 1* croissance *du* mycélium *; 2* croissance *et sporulation* | | | | | | |

La sporulation de la souche MUCL 45632 est très intense à 20°C, ce qui correspond à son optimum de croissance. A 14 °C, son développement est correct mais sa sporulation n'est pas à son maximum. A 4 °C, le mycélium ne s'est pas développé mais il n'est pas mort. En effet, une fois le bouchon repiqué sur PDA et mis à incuber à 20 °C, ce dernier repart très bien. Par contre à 37 °C, le bouchon mycélien ne repart pas ce qui signifie que le champignon MUCL 45632 est mort. Le manque de viabilité du mycélium à 37 °C n'est pas gênant car les champignons pathogènes cibles ne se développent pas à cette température.

La souche MUCL 45632 se comporte mieux à basse température que les autres souches testées. La souche MUCL 45632 a été retenue pour sa capacité à se développer et à agir dès que les températures remontent au printemps. La plage de température active de la souche MUCL 45632 est assez large pour couvrir la période de croissance des plantes et assurer une protection pendant les périodes à risques.

### Etude de la stimulation de la croissance des plantes.

La souche *Trichoderma atroviride* MUCL 45632 qui se multiplie dans le sol, à proximité ou en symbiose avec les racines possède des propriétés de stimulation de la croissance des plantes. Cela a été démontré par plusieurs essais sur différentes plantes.

### A. Stimulation de croissance des concombres (premier exemple) :

Culture de concombre *Cucumis sativa*, variété « Vert long des maraîchers » en tube conique de 50 ml sur perlite expansée autoclavée sous rampes lumineuses de 6000 lux pendant 21 jours. La préparation de *Trichoderma* MUCL 45632 (10⁹ spores/g) est apportée dans la Perlite aux doses de 1 % (vol.) et de 3 % (vol.). Le poids frais et le poids sec des racines ont été mesurés.

Les résultats sont rapportés dans le tableau ci dessous (valeurs calculées par rapport au témoin):

| **modalités** | poids frais par plant | coefficient de variation du poids frais | poids sec par plant | coefficient de variation du poids sec |
|---|---|---|---|---|
| **MUCL 45632 1 %** | *370 mg* | *3,6* | *15,4 mg* | *2,6* |
| ***MUCL 45632* 3 %** | *380 mg* | *3,7* | *16,5 mg* | *2,8* |
| **Témoin non traité** | *100 mg* | | *5,9 mg* | - |

L'augmentation de poids sec, d'après le tableau ci-dessus, prouve qu'il y a eu une augmentation de la production de biomasse.

En outre, à la dose 1 %, le poids frais des racines est multiplié par 2,7 par rapport au témoin et le poids sec des mêmes racines par 1,6. Pour la dose 3 % les valeurs obtenues sont supérieures démontrant l'efficacité de la souche.

### B. Stimulation de croissance des concombres (second exemple) :

Culture de concombre *Cucumis sativa*, variété « Marketer » en pot sur terreau dans un Phytotron. L'intensité lumineuse est de 10000 lux, la température de 22 °C et la photopériode de 16 h pendant 45 jours. La préparation de *Trichoderma* MUCL 45632 (10⁹ spores/g) est apporté au terreau à la dose de 2 % (vol.). Le poids frais des racines et des parties aériennes sont mesurés.

Les résultats sont rapportés dans le tableau ci dessous (valeur calculée par rapport au témoin):

| **modalités** | Racines poids frais par plant | variation | Partie aérienne poids frais par plant | variation |
|---|---|---|---|---|
| ***MUCL 45632 2%*** | *9,6 g* | *35,2 %* | *7,5 g* | *15,4 %* |
| **Témoin non traité** | *7,1 g* | - | *6,5 g* | - |

La stimulation de croissance à la dose de 2 % est importante au niveau des racines avec 35,2 % d'augmentation. Au niveau des parties aériennes elle est de 15,4 %.

### C. Stimulation de croissance des laitues :

Culture de salade *Lactucasativa*, variété « Grand Rapids » sur plaque multipots sur terreau. L'intensité lumineuse est de 3000 lux, la température de 25 °C et la photopériode de 16 h pendant 21 jours. La préparation de *Trichoderma* MUCL 45632 (10⁹ spores/g) est apportée au terreau à la dose de 5 % (vol.). Le poids frais et le poids sec des parties aériennes ont été mesurés.

Les résultats sont rapportés dans le tableau ci dessous (valeurs calculées par rapport au témoin):

| modalités | poids frais par plant | coefficient de variation du poids frais | poids sec par plant | coefficient de variation du poids sec |
|---|---|---|---|---|
| **MUCL 45632 5%** | *144,6 mg* | *2,0* | *8,4 mg* | *2,1* |
| **Témoin non traité** | *72,5 mg* | - | *4,0 mg* | - |

La stimulation de croissance à la dose de 5 % est efficace. Les coefficients d'augmentation de matière sont élevés démontrant une excellente stimulation de croissance de la part de *Trichoderma* MUCL 45632. L'observation du poids sec montre que ce qui a été crée est de la biomasse. *Trichoderma* MUCL 45632 n'a pas induit une turgescence des cellules végétales mais bien une multiplication organisée de cellules végétales.

### D. Stimulation de croissance du riz :

Culture de riz *Oriza sativa*, variété « Ruille » en tube conique de 50 ml sur Perlite autoclavée pendant 15 jours. L'intensité lumineuse est de 6000 lux, la température de 25 °C et la photopériode de 16 h. La préparation de *Trichoderma* MUCL 45632 (10⁹ spores/g) est apporté dans la perlite expansée à la dose de 1 % (vol.). Le poids frais et le poids sec des tiges ont été mesurés.

Les résultats sont rapportés dans le tableau ci dessous (valeurs calculées par rapport au témoin) :

| modalités | poids frais par plant | coefficient de variation du poids frais | poids sec par plant | coefficient de variation du poids sec |
|---|---|---|---|---|
| ***MUCL 45632 1%*** | *43,7 mg* | *2,0* | *6,0 mg* | *2,0* |
| **Témoin non traité** | *22,1 mg* | - | *3,0 mg* | - |

L'augmentation de poids frais s'accompagne de l'augmentation de matière sèche preuve que *Trichoderma atroviride* MUCL 45632 a favorisé la production de biomasse du riz en culture submergée.

### E. Stimulation de croissance de tomates (premier exemple) :

Culture de tomates *Solanum lycopersicum,* variété « Saint Pierre » en pot (7 x 7 x 6 cm) sur terreau. L'intensité lumineuse est de 10000 lux, la température de 22 °C et la photopériode de 16 h pendant 45 jours. La préparation de *Trichoderma* MUCL 45632 (10⁹ spores/g) est apportée avec le terreau à la dose de 2 % (vol.). Le poids frais et le poids sec des tiges ont été mesurés.

Les résultats sont rapportés dans le tableau ci dessous (valeurs calculées par rapport au témoin) :

| | Racines | | Partie aérienne | |
|---|---|---|---|---|
| modalités | poids frais par plant | variation | poids sec par plant | variation |
| ***MUCL 45632 2%*** | *1,88 g* | *34 %* | *4,12 g* | *60 %* |
| **Témoin non traité** | *1,40 g* | | *2,58 g* | - |

Une fois encore, il apparaît une augmentation du poids frais aussi bien sur les racines que sur les parties aériennes grâce à l'action du *Trichoderma* MUCL 45632.

### F. Stimulation de croissance de tomates (second exemple) :

Culture de tomates *Solanum lycopersicum,* variété « Saint Pierre » en tube conique de 50 ml sur Perlite autoclavée pendant 1 mois. L'intensité lumineuse est de 6 000 lux, la température de 25 °C et la photopériode de 16 h. La préparation de *Trichoderma* MUCL 45632 (10⁹ spores/g) est apportée dans la Perlite à la dose de 3 % (vol.). Le poids frais et le poids sec des racines et des parties aériennes ont été mesurés.

Pour les racines, les résultats sont rapportés dans le tableau ci dessous (valeurs calculées par rapport au témoin) :

| modalités | poids frais des racines par plant | coefficient de variation du poids frais | poids sec des racines par plant | coefficient de variation du poids sec |
|---|---|---|---|---|
| ***MUCL 45632* 3%** | *117,7 mg* | *9,1* | *7,6 mg* | *9,5* |
| **Témoin non traité** | *12,9 mg* | - | *0,8 mg* | - |

Pour les parties aériennes, les résultats sont rapportés dans le tableau ci dessous (valeurs calculées par rapport au témoin) :

| modalités | poids frais des parties aériennes par plant | coefficient de variation du poids frais | poids sec des parties aériennes par plant | coefficient de variation du poids sec |
|---|---|---|---|---|
| ***MUCL 45632* 3%** | *170,3 mg* | *8,5* | *14,7 mg* | *7,7* |
| **Témoin non traité** | *20,1 mg* | - | *1,9 mg* | - |

Les résultats de cette expérience démontrent une fois de plus l'effet stimulateur de la souche *Trichoderma* MUCL 45632. Les plants obtenus sont plus vigoureux avec un chevelu racinaire très étoffé et des parties aériennes plus denses.

### Conclusion :

L'effet stimulateur de *Trichoderma* MUCL 45632 a été mis en évidence dans les 6 exemples précédents et cela aussi bien sur les racines que les parties aériennes. Ce champignon stimule significativement la synthèse de biomasse par la plante qui se caractérise par une amélioration de la croissance, une meilleure précocité et un meilleur rendement.

Comme mentionné précédemment, *Trichoderma atroviride* MUCL 45632 possèdeégalement des propriétés antagonistes à l'égard de champignons pathogènes et constitue donc un bon agent de lutte biologique.

### Etude comparative de l'effet stimulateur des différents Trichoderma sur la croissance des plantes.

Certaines souches de champignons du sol *Trichoderma* sont connues pour favoriser la croissance des plantes et d'autres pour la défavoriser. L'expression de propriétés stimulatrices ou inhibitrices n'est pas caractéristique du genre *Trichoderma,* ni d'une espèce particulière dans ce genre. Ce sont des variétés au sein de certaines espèces qui peuvent se montrer plus ou moins favorables ou défavorables à la croissance des plantes ou au développement des racines.

L'identification des espèces par les critères morphologiques n'est pas fiable car les *Trichoderma* sont très polymorphes. Pour les identifier avec certitude, il faut utiliser une méthode moléculaire communément admise à partir de 1998, le séquençage des ITS-1 et ITS-2 (internai transcribed spacers) de l'ADN ribosomal (Gams W., Meyer W., Mycologia, 90 (5), 904-915, 1998). Cette méthode permet l'identification par comparaison des séquences ITS-1 et 2 avec celles de souches de référence déposées dans des collections publiques, par exemple les souches ATCC 28036 (ITSs : Genbank Z48817) et ATCC 36042 (ITSs : Genbank Z48812) pour *Trichoderma atroviride.*

La souche de *Trichoderma atroviride* MUCL 45632, ainsi que d'autres souches que l'on peut trouver dans les collections et dans les produits commerciaux ont été identifiées par des techniques biochimiques et moléculaires, puis comparées entre elles. La souche MUCL 45632 est une variété de *Trichoderma. atroviride* dont les propriétés stimulatrices de croissance sont particulièrement intenses. L'exemple suivant montre que la souche MUCL 45632 possède des propriétés de stimulation de la croissance des plantes supérieures à celles d'autres *Trichoderma atroviride* et d'autres espèces de *Trichoderma.*

Dans cet exemple, les souches de *Trichoderma* étudiées sont :
- MUCL 45632 (*T. atroviride*, identifiée par séquençage des ITS-1 et ITS-2 de l'ADNr et caractérisée à l'aide de tests biochimiques ; souche isolée par la demanderesse d'un sol en friche)
- Nixe 00401A (*T. atroviride*, identifiée par séquençage des ITS-1 et ITS-2, souche isolée par la demanderesse d'une culture de champignons *Pleurotus ostreatus*)
- CNCM I-1571 (*T*. *atroviride,* identifiée par séquençage des ITS-1 et ITS-2, Brevet FR2780972, présentée comme une souche produisant des alkyl-gamma-lactones qui stimulent le développement végétal)
- T-22 ou 1295-22 (ATCC 20847) (*T*. *harzianum* Rifai, identifiée par séquençage des ITS-1 et ITS-2, Brevet EP0285987, distribuée sous le nom Trianum P par Koppert BV, Pays-Bas, connue pour ses propriétés stimulatrices du développement des racines, *cf.* Harman G.E. et al., Nature Reviews / Microbiology, 2, 43-56, 2004)
- T-39 (CNCM I-952) (*Trichoderma harzianum* Rifai, identifiée par séquençage des ITS-1 et ITS-2, Brevet EP0466133)
- TV1 (Nixe 13602A) (*T*. *harzianum,* identifiée par séquençage des ITS-1 et ITS-2, souche de produit commercial Agribiotec, Italie)
- Nixe 30301E (*Trichoderma* sp., identifiée morphologiquement mais pas génétiquement, souche isolée par la demanderesse d'une culture hors-sol *d'Allium porum*)

L'étude est réalisée sur une culture de concombre *Cucumis sativa* variété « Marketer ». Dans chaque tube conique de 50 mL, on place 1 graine sur 45 mL de perlite expansée autoclavée. Les plantes ne reçoivent aucun apport d'engrais. L'éclairage de 10000 lux avec une photopériode de 16 heures est obtenu par des lampes fluorescentes. La température varie entre 20 et 25 °C. La culture dure 21 jours.

Les champignons d'essai sont cultivés sur PDA. Les spores sont collectées dans de l'eau stérile et sont comptées sur cellule de Malassez. Les spores de *Trichoderma* sont apportées à la dose de 2,2.10⁸ spores pour 45 mL de perlite, soit 5.10⁶ spores/mL de support de culture. Les tubes sont arrosés avec 28 mL d'eau stérile au départ, puis l'eau est apportée selon les besoins.

A la fin de la culture, l'aspect des plantes, les poids frais et les poids sec des parties aériennes et des racines sont mesurés.

| | Observations après 21 jours de culture | | | | | | |
|---|---|---|---|---|---|---|---|
| Modalités | Graines germées % | Plants flétris % | Plants normaux % | Nombre moyen de feuilles par plant (1) | Longueur moyenne des tiges cm | Indice de biomasse fraîche moyenne / témoin NT (2) | Indice de biomasse sèche moyenne / témoin NT (3) |
| *T. atroviride MUCL 45632* | *100* | *0* | *100* | *2* | *3,5* | *1,9* | *1,4* |
| *T. atroviride Nixe 00401A* | *75* | *0* | *75* | *1* | *2,7* | *1,4* | *1,2* |
| *T. atroviride I-1571* | *75* | *0* | *75* | *0,33* | *2,5* | *0,8* | *0,7* |
| *T. harzianum T-22* / *A TCC 20847* | *100* | *0* | *100* | *1* | *3,0* | *1,2* | *0,9* |
| *T. harzianum T-39* / *I-952* | *75* | *0* | *75* | *1* | *3,1* | *1,0* | *0,9* |
| *T. harzianum TV1* / *Nixe 13602A* | *100* | *25* | *75* | *0,33* | *2,8* | *0,9* | *0,9* |
| *Trichoderma* sp. *Nixe 30301E* | *100* | *0* | *100* | *1* | *2,4* | *0,7* | *0,6* |
| *Plantes témoins non traitées (NT)* | *75* | *0* | *75* | *1* | *2,7* | *1,0* | *1,0* |

| | | | | | | | |
|---|---|---|---|---|---|---|---|
| Notes : (1) nombre moyen de feuilles vraies par plant, cotylédons non compris ; (2) rapport entre le poids moyen des plantes fraîches collectées (racines comprises) et le poids moyen des plantes témoins non traitées (NT) ; (3) rapport entre le poids moyen des plantes sèches collectées (racines comprises) et le poids moyen des plantes témoins non traitées (NT) | | | | | | | |

La germination des graines n'est pas gênée par la souche MUCL 45632. Dans cet essai, il est difficile de savoir si les variations de taux de germination proviennent des graines ou de l'effet des *Trichoderma.* Il a été observé qu'une concentration excessive en *Trichoderma* provoque une destruction des graines, mais ce n'est pas le cas dans ce dispositif.

La culture ne comprend aucune fertilisation afin de mettre en évidence les effets stimulateurs liés à l'interaction entre la plante et le champignon. Le substrat de culture est totalement inerte et n'interfère pas dans le processus de stimulation de croissance. Les ressources nutritives proviennent intégralement de la graine et n'ont aucun impact sur ledit processus.

Les plants traités par MUCL 45632 se développent plus rapidement que les autres avec plus de feuilles et une plus grande taille. Le *T. atroviride* I-1571 et le *T*. *harzianum* T-22, pourtant connus pour leurs effets favorables, sont moins efficaces que MUCL 45632. Après trois semaines, les plants traités par MUCL 45632 possèdent au moins une semaine d'avance sur les plants non traités. Ils se sont développés harmonieusement, sans élongation anormale.

Un autre aspect caractéristique de l'effet stimulateur est l'augmentation de biomasse obtenue à l'aide de MUCL 45632. La production moyenne de biomasse fraîche par plant est 1,9 fois supérieure à celle d'un plant moyen non traité malgré l'absence d'apport de nutriments extérieurs. La production de biomasse sèche suit la même tendance. L'écart est encore plus important par comparaison directe avec les plants traités par *T. atroviride* I-1571. L'effet de la souche de *T*. *atroviride* Nixe 00401A est bon mais il reste inférieur à celui de MUCL 45632. On notera l'effet dépressif des souches *T*. *atroviride* I-1571 et *Trichoderma* sp. Nixe 30301E.

L'effet stimulateur de *Trichoderma atroviride* MUCL 45632 est supérieur en tous points à celui d'autres *Trichoderma,* aussi bien sur les racines que les parties aériennes. Le champignon stimule significativement la synthèse de biomasse par la plante qui se caractérise par une amélioration de la croissance, une meilleure précocité et un meilleur rendement. Les propriétés remarquables de la variété MUCL 45632 de *T. atroviride* sont confirmées.

### Propriétés antagonistes

### Essais in vitro :

La souche MUCL 45632 a fait l'objet de tests au laboratoire contre divers champignons pathogènes du sol et des parties aériennes. Ces essais ont été faits à différentes températures dans la plage de développement de la souche MUCL 45632.

Dans une boite de Pétri gélosée au PDA, on place à la périphérie un bouchon mycélien de la souche de *Trichoderma atroviride* MUCL 45632 et à l'opposé, un bouchon mycélien du champignon pathogène. Après 15 jours de culture à la température choisie, le comportement de la souche de *Trichoderma* face au pathogène est analysé sur la base des critères : mycélium recouvert ou pas, avec ou sans sporulation. La viabilité du bouchon du pathogène est analysée en le prélevant, en le déposant sur une boîte PDA et en le cultivant à 25 °C. Cela permet de déterminer si, après confrontation avec *Trichoderma,* il est toujours capable de donner naissance à un mycélium ou s'il a été entièrement détruit.

### Résultat des tests de compétition in vitro contre différents champignons pathogènes :

| Champignons pathogènes | 15 °C | 25 °C | 30 °C | >30 °C |
|---|---|---|---|---|
| *Aspergillus carbonarius* | *n.d.* | *détruit* | *détruit* | *détruit* |
| *Botrytis cinerea* | *détruit* | *détruit* | *détruit* | *détruit* |
| *Fusarium oxysporum* | *n.d.* | *détruit* | *détruit* | *détruit* |
| *Phytophthora infestans* | *n.d.* | *détruit* | *détruit* | *détruit* |
| *Sclerotinia minor* | *détruit* | *détruit* | *détruit* | *détruit* |
| *Sclerotinia sclerotiorum* | *détruit* | *détruit* | *détruit* | *détruit* |

| | | | | |
|---|---|---|---|---|
| *n.d. : non déterminé* | | | | |

La souche est remarquable dans le sens où, à partir de 15 °C, elle tue *Botrytis cinerea, Sclerotinia minor* et *Sclerotinia sclerotiorum* qui sont des pathogènes actifs à basse température.

Pour *Aspergillus carbonarius, Fusarium oxysporum* et *Phytophthora infestans,* la souche MUCL 45632 les éradique à leur température optimale de croissance entre 25 et 30 °C.

Les propriétés antagonistes destinées à lutter spécifiquement contre les maladies du bois de la vigne ont été testées. Pour ce faire les tests de compétition suivants ont été réalisés :
La souche MUCL 45632 a été confrontée *in vitro* contre divers champignons pathogènes des maladies du bois de la vigne (ITV Nîmes France).
- Esca : *Phaeomoniella chlamydospora* (Pc LR47 et Pc LR 81), *Phaeoacremonium aleophilum* (Pa LR3 et Pa LR23) et *Fomitiporia punctata* (Fp LR14 et Fp LR30).
- Eutypiose : *Eutypa lata* (Elcc260 et El MT 247)
- Dépérissement de la syrah : *Botryosphaeria obtusa* (Bo F98-1 et Bo F99-8)
- Black Dead Arm : *Botryosphaeria parva* (Bd 021)
- Greffés-soudés : *Botryosphaeria stevensii* (Bs 001)

Dans une boite de Pétri gélosée au Malt-Agar, on place à la périphérie un bouchon mycélien de la souche de *Trichoderma atroviride* MUCL 45632 et à l'opposé, un bouchon mycélien du champignon pathogène. Trois répétitions sont faites pour chaque test. Les boîtes de Pétri sont cultivées à 25 °C. La croissance du champignon pathogène seul sert de témoin. La distance d'inhibition I, exprimée en mm, correspond à la différence entre le rayon moyen R1 de la colonie de la boîte témoin et le rayon moyen R2 qui est situé sur là droite reliant le centre des deux protagonistes en confrontation (I = R1 - R2).

Le taux d'inhibition est calculé au bout de :
- 4 jours pour *Botryosphaeria parva* (Bd 021),
- 5 jours pour *Botryosphaeria obtusa* (Bo F98-1 et Bo F99-8) et *Botryosphaeria stevensii* (Bs 001),
- 7 jours pour *Eutypa lata* (Elcc260 et El MT 247) et *Fomitiporia punctata* (Fp LR14 et Fp LR30),
- 9 jours pour *Phaeomoniella chlamydospora* (Pc LR47 et Pc LR 81),
- 12 jours pour *Phaeoacremonium aleophilum* (Pa LR3 et Pa LR23).

Le comportement de la souche de *Trichoderma* face au pathogène est analysé sur la base des critères de recouvrement du mycélium, de sporulation et de zone de mélanisation.

La viabilité du bouchon du pathogène après essai est analysée en le prélevant, en le déposant sur une boîte Malt-Agar et en le cultivant à 25 °C. Cela permet de déterminer si, après confrontation avec *Trichoderma*, il est toujours viable ou a été détruit.

La souche MUCL 45632 est comparée à la souche de *Trichoderma* I-3151 qui a subi les mêmes tests dont les résultats sont publiés dans le brevet FR2864832.

### Résultat des tests de confrontation in vitro contre différents champignons pathogènes du bois de la vigne :

| | Distance d'inhibition (mm) | |
|---|---|---|
| Champignon pathogène | MUCL 45632 | I-3151 |
| *Phaeomoniella chlamydospora Pc LR47* | *4*,*3* | *3* |
| *Phaeomoniella chlamydospora Pc LR81* | *2*,*4* | *4,2* |
| *Phaeoacremonium aleophilum Pa LR3* | *7*,*5* | *10,7* |
| *Phaeoacremonium aleophilum Pa LR23* | *1,9* | *3,5* |
| *Fomitiporia punctata Fp LR14* | *7,3* | *7,7* |
| *Fomitiporia punctata Fp LR30* | *9,7* | *10,7* |
| *Eutypa lata Elcc260* | *5* | *9* |
| *Eutypa lata EI MT 247* | *10,4* | *12* |
| *Botryosphaeria obtusa Bo F98-1* | *29,2* | *28,2* |
| *Botryosphaeria obtusa Bo F99-8* | *25*,*2* | *24*,*6* |
| *Botryosphaeria parva Bd 021* | *23,9* | *21*,*7* |
| *Botryosphaeria stevensii Bs 001* | *35,7* | *32,7* |

La souche MUCL 45632 présente une efficacité incontestable contre les maladies dues aux *Botryosphaeria* : dépérissement de la syrah, Black Dead Arm et greffés-soudés. Les diamètres d'inhibition sont élevés, démontrant ainsi l'action positive de MUCL 45632. A l'observation binoculaire, elle recouvre entièrement les pathogènes et fructifie en les tuant irrémédiablement.

Contre l'esca de *Phaeomoniella chlamydospora* et de *Fomitiporia punctata,* elle montre une bonne efficacité car elle recouvre entièrement les pathogènes et fructifie en les tuant complètement. Son efficacité est limitée contre l'esca due aux souches Pa LR3 et Pa LR23, dans le sens où elle inhibe la croissance des pathogènes mais ne les tue pas, de la même manière que la souche I-3151.

Contre l'eutypiose (Elcc260 et EI MT 247), la souche MUCL 45632 montre des diamètres d'inhibitions acceptables.

En conclusion, la souche *Trichoderma atroviride* MUCL 45632 possède une bonne capacité de lutte contre les maladies du bois : elle est très efficace contre le dépérissement de la syrah, le Black Dead Arm et les greffés-soudés et équivalente à la souche *Trichoderma* I-3151 dans le cas des autres maladies.

### Essais en champ et sous serre :

### A. Etude sur radis (Raphanus sativus) contre Fusarium oxysporum et Rhizoctonia solani du sol.

Le produit formulé *Trichoderma atroviride* MUCL 45632 (10⁹ spores/g) a été appliqué selon deux modalités sur une culture de radis dans un sol infectée par F. *oxysporum* et *R. solani.* Le nombre de plantes est 130 000 sur 500 m². Dans cet essai, le *Trichoderma* est apporté par aspersion.

| Produit testé | Modalité | Plantes malades |
|---|---|---|
| Préparation MUCL 45632 | 1 application 2 g/m² au semis et 1 application 1 g/m² à 15 jours | 24 % |
| Préparation MUCL 45632 | 1 application 2 g/m² au semis et 2 applications 2 g/m² espacées de 7 j. | 0 % |
| Traitement conventionnel Laicon-L (Polyoxin B) | conditions standard | 0 % |

Le traitement par la préparation de *Trichoderma atroviride* MUCL 45632 offre une protection comparable à celle du produit antibiotique Laicon-L (Polyoxin B).

### B. Essai sur tomate (Solanum lycopersicum) sous serre contre Botrytis cinerea.

Le produit formulé *Trichoderma atroviride* MUCL 45632 (10⁹ spores/g) a été appliqué sur des plants de tomates dont 4 feuilles ont été sectionnées. Après séchage de l'application de *Trichoderma,* les plantes sont infectées artificiellement par une suspension de *Botrytis cinerea.* Le nombre de plantes est 48 (4 parcelles de 12). L'évolution de la maladie sur les plaies de section des feuilles est observée après 13 jours.

| Produit testé | Modalité | Indice d'infection plaies à l'air libre | Indice d'infection plaies enveloppées |
|---|---|---|---|
| Préparation MUCL 45632 | 1 pulvérisation à 1 g/m² | 1,07/4 | 2,65/4 |
| Traitement conventionnel Scala (Pyrimethanil) | 1 pulvérisation à 0,2 mL/m² | 1,14/4 | 2,78/4 |
| Témoin non traité | | 2,30/4 | 3,65/4 |

Le traitement par la préparation de *Trichoderma* MUCL 45632 offre une protection comparable à celle du traitement chimique conventionnel.

Lorsque les plaies de taille sont à l'air libre, cette protection est bonne.

### C. Essai sur piment (Capsicum annuum) sous serre contre Phytophthora capsici.

Le produit formulé *Trichoderma atroviride* MUCL 45632 (10⁹ spores/g) a été appliqué sur les plants de piment 1 mois après le semis en février puis tous les 15 à 30 jours, jusqu'au mois d'août. Les semis ont été faits sous tunnel en terrain désinfecté et les jeunes plantes ont été transplantées en champ en mai. Le nombre de plantes est 1800 (environ 800 m²). L'infection par *Phytophthora capsici* est naturelle. Les symptômes de la maladie sont mesurés dès leur apparition au mois d'août.

Le traitement par la préparation de *Trichoderma* MUCL 45632 réduit significativement le taux de plantes malades.

D'autres aspects de l'invention, en particulier d'autres utilisations du *Trichoderma atroviride* selon l'invention ou de ses produits dérivés, seront apparents à l'homme du métier et sont compris dans l'étendue de la protection.

## Revendications

1. Souche de *Trichoderma atroviride* **caractérisée en ce qu'**elle possède le profil biochimique, morphologique et physiologique suivant :
| Milieu de culture | Vitesse de croissance | Aspect de la sporulation |
|---|---|---|
| (1) milieu de référence PDA à 25°C potato dextrose agar 39 g/L | très rapide - apparition d'un thalle ras translucide en deux jours environ | vert intense |
| Milieu de culture | Vitesse de croissance par rapport au milieu PDA | Aspect de la sporulation par rapport au milieu PDA |
|---|---|---|
| (2) milieu minimum à 4°C glucose 5 g/L + peptone 5 g/L + Mg SO₄, 7 H₂O 0,5 g/L + KH₂PO₄ 1 g/L + rose bengale 25 mg/L + agar 15 g/L | très lente | absente |
| (3) milieu minimum à 25°C glucose 5 g/L + peptone 5 g/L + Mg SO₄, 7 H₂O 0,5 g/L + KH₂PO₄ 1 g/L + rose bengale 25 mg/L + agar 15 g/L | très légèrement ralentie | intense verte à beige |
| (4) milieu minimum à | pas de croissance | pas de sporulation |
| 37°C glucose 5 g/L + peptone 5 g/L + Mg SO₄, 7 H₂O 0,5 g/L + KH₂PO₄ 1 g/L + rose bengale 25 mg/L + agar 15 g/L | | |
| (5) milieu minimum 1% glucose à 25 °C le glucose à 5 g/L du milieu minimum est augmenté à 10 g/L | ralentie | plus faible |
| (6) milieu minimum 1 % éthanol à 25 °C le glucose à 5 g/L du milieu minimum est remplacé par de l'éthanol à 10 mL/L | mycélium plus étendu mais moins dense | plus faible |
| (7) milieu minimum 1 % acide citrique à 25 °C le glucose à 5 g/L du milieu minimum est remplacé par de l'acide citrique à 10 g/L | mycélium moins dense | absente |
| (8) milieu minimum 1 % acide lactique à 25 °C le glucose à 5 g/L du milieu minimum est remplacé par de l'acide lactique à 10 g/L | légèrement ralentie | plus faible |
| (9) milieu minimum 0,2 % glycine à 25 °C la peptone à 5 g/L du milieu minimum est remplacée par de la glycine à 2 g/L | légèrement ralentie | plus faible |
| (10) milieu minimum 0,2 % oxalate d'ammonium | légèrement ralentie | inchangée |
| à 25 °C la peptone à 5 g/L du milieu minimum est remplacée par de l'oxalate d'ammonium à 2 g/L | | |
| (11) milieu minimum 1 % oxalate d'ammonium à 25 °C la peptone à 5 g/L du milieu minimum est remplacée par de l'oxalate d'ammonium à 10 g/L | très ralentie | absente |
| (12) milieu minimum 0,2% urée à 25 °C la peptone à 5 g/L du milieu minimum est remplacée par de l'urée à 2 g/L | mycélium plus étendu mais moins dense | plus faible |
| (13) milieu minimum 0,2% nitrite de sodium à 25 °C la peptone à 5 g/L du milieu minimum est remplacée par du nitrite de sodium à 2 g/L | mycélium plus étendu mais moins dense | inchangée |
| (14) milieu minimum à 10 mg/l cristal violet à 25 °C | très ralentie | inchangée |
| (15) milieu minimum à 50 mg/l cristal violet à 25 °C | très ralentie | absente |
| (16) milieu malt-sulfate de cuivre à 25 °C à la lumière malt extract 10 g/L + CuSO₄, 5 H₂O 200 mg/L + agar 15 g/L | mycélium moins dense | inchangée |
| (17) milieu malt-sulfate de cuivre à 25 °C à l'obscurité malt extract 10 g/L + CuSO₄, 5 H₂O 200 mg/L + agar 15 g/L | mycélium moins dense | inchangée |
| (18) milieu Czapek-ammonium à 25 °C sucrose 30 g/L + NaNO₃ 3 g/L + K₂HPO₄ 1 g/L + MgSO₄, 7 H₂O 0,5 g/L + KCI 0,5 g/L + FeSO₄, 7 H₂O 10 mg/L + (NH₄)₂SO₄ 5 g/L + agar 15 g/L | mycélium plus étendu moins dense en zone centrale | un peu plus faible |
| (19) milieu nitrito-saccharose à 25 °C saccharose 10 g/L + NaNO₂ 2 g/L + agar 15 g/L | mycélium beaucoup moins dense | beaucoup plus faible |
| (20) milieu glycérol-nitrate à 25 °C glycérol 5 ml/L: + NaNO₃ 3g/L + agar 15 g/L | mycélium beaucoup moins dense | beaucoup plus faible |
ladite souche possédant en outre les aspects culturaux suivants :
- sur milieu PDA (Potato Dextrose Agar), il apparaît rapidement, en deux jours environ, un thalle ras translucide qui devient blanc le troisième jour avec l'apparition des conidiophores ; ce mycélium ras vire au vert très sombre au bout de cinq jours, signe d'une sporulation intense et le revers de la culture est incolore ;
- sur milieu MA (Malt Agar), son développement est légèrement différent et caractéristique de l'espèce *Trichoderma atroviride,* le mycélium forme des cercles concentriques de sporulation vert intense autour du point d'inoculation avec un thalle qui reste translucide jusqu'à la fin de la culture ; l'aspect microscopique est le même sur les deux milieux, les conidiophores sont très ramifiés avec des cellules conidiogènes (phialides) disposées en verticille par 2 ou 3, lesdites phialides sont ampulliformes, droites ou incurvées, de dimensions 2,5 à 3 µm par 8 à 14 µm et les conidies réunies au sommet de la phialide, sont globuleuses à paroi lisse, de couleur verte et mesurent environ 3 µm de diamètre ;
- ladite souche se développe entre 12°C et 36°C avec un optimum de croissance entre 20°C et 25°C sur les milieux gélosés classiques de type PDA, MA, OA (Oat Meal Agar) ;
les séquences nucléotidiques espaceurs internes transcrits 1 et 2 de ladite souche étant identiques à celles de la souche *Trichoderma atroviride* ATCC 36042.

2. Milieu de culture **caractérisé en ce qu'**il comprend au moins un *Trichoderma atroviride* selon la revendication 1 et au moins un substrat, particulièrement un substrat d'origine végétale et plus particulièrement un substrat riche en polyholosides tels que l'amidon, l'hémicellulose ou la cellulose.

3. Milieu de culture selon la revendication 2 **caractérisé en ce qu'**il comprend au moins un diluant minéral inerte.

4. Milieu de culture selon la revendication 3 **caractérisé en ce que** ledit diluant est spécifiquement sélectionné pour diluer et/ou aérer ledit milieu de culture, par exemple, ledit diluant est choisi dans le groupe constitué par les argiles calcinées en grains telles que l'attapulgite, les billes d'argile soufflées, les poudres de roches volcaniques comme la pouzzolane ou la pierre ponce, la perlite expansée, la vermiculite expansée et la laine de roche.

5. Milieu de culture selon l'une des revendications 2 à 4 **caractérisé en ce qu'**il comprend au moins un élément minéral utile à la croissance de la souche de *Trichoderma atroviride* selon la revendication 1, tel qu'un nitrate, de l'ammonium, un orthophosphate, du potassium, du magnésium, du calcium ou un oligo-élément.

6. Milieu de culture selon l'une des revendications 2 à 5 **caractérisé en ce qu'**il comprend un acide, de préférence minéral, permettant de maintenir le pH du milieu entre 2 et 4 et de préférence entre 2,7 et 3,3.

7. Concentré déshydraté d'un *Trichoderma atroviride* selon la revendication 1, particulièrement sous forme de poudre.

8. Procédé d'obtention d'une poudre à base du *Trichoderma atroviride* selon la revendication 1, **caractérisé en ce qu'**il comprend les étapes de fermentation du milieu de culture selon l'une des revendications 4 à 6, de déshydratation à basse température, suivies d'une étape de broyage.

9. Utilisation d'un *Trichoderma atroviride* selon la revendication 1 comme stimulant de la germination et/ou de la croissance des plantes.

10. Utilisation d'un *Trichoderma atroviride* selon la revendication 1 en tant qu'agent de biocontrôle, notamment contre les champignons pathogènes des plantes.

11. Procédé d'isolement d'une souche de *Trichoderma atroviride* selon la revendication 1, **caractérisé en ce que** ledit procédé comprend les étapes successives suivantes :
a) Prélèvement d'un extrait de champignon *Trichoderma atroviride* dans un milieu adéquat comme un sol inculte ou cultivé, sur les plantes ou dans les bois en décomposition issus de ceps de vigne, de branches ou de troncs d'arbres,
b) Dilution de l'extrait prélevé avec de l'eau stérile,
c) Culture de cet extrait sur au moins un milieu sélectif, ledit milieu sélectif étant compris dans le groupe constitué par : le milieu MA2 (malt agar 2%) et le milieu RB-S-F (McFadden et Sutton),
d) Repiquage des champignons *Trichoderma atroviride* issus de spores isolées dans des boîtes de Pétri contenant du milieu PDA (Potato Dextrose Agar),
e) Culture des champignons *Trichoderma atroviride* repiqués durant douze jours dans les conditions de culture suivantes :
- (1) milieu PDA à 25 °C, à 39 g/L;
- (2) milieu minimum à 4 °C, comprenant du glucose à 5 g/L, de la peptone à 5 g/L, du Mg SO₄, 7 H₂O à 0,5 g/L, du KH₂PO₄ à 1 g/L, du rose bengale à 25 mg/L, de l'agar à 15 g/L;
- (3) milieu minimum à 25 °C, de composition identique à celle du milieu minimum (2);
- (4) milieu minimum à 37 °C, de composition identique à celle du milieu minimum (2);
- (5) milieu minimum 1 % glucose à 25 °C, le glucose à 5 g/L du milieu minimum étant augmenté à 10 g/L;
- (6) milieu minimum 1 % éthanol à 25 °C, le glucose à 5 g/L du milieu minimum étant remplacé par de l'éthanol à 10 mL/L;
- (7) milieu minimum 1 % acide citrique à 25 °C, le glucose à 5 g/L du milieu minimum étant remplacé par de l'acide citrique à 10 g/L;
- (8) milieu minimum 1 % acide lactique à 25 °C, le glucose à 5 g/L du milieu minimum étant remplacé par de l'acide lactique à 10 g/L;
- (9) milieu minimum 0,2 % glycine à 25 °C, la peptone à 5 g/L du milieu minimum étant remplacée par de la glycine à 2 g/L;
- (10) milieu minimum 0,2 % oxalate d'ammonium à 25 °C, la peptone à 5 g/L du milieu minimum étant remplacée par de l'oxalate d'ammonium à 2 g/L;
- (11) milieu minimum 1 % oxalate d'ammonium à 25 °C, la peptone à 5 g/L du milieu minimum étant remplacée par de l'oxalate d'ammonium à 10 g/L;
- (12) milieu minimum 0,2 % d'urée à 25 °C, la peptone à 5 g/L du milieu minimum étant remplacée par de l'urée à 2 g/L;
- (13) milieu minimum 0,2 % nitrite de sodium à 25 °C, la peptone à 5 g/L du milieu minimum étant remplacée par du nitrite de sodium à 2 g/L;
- (14) milieu minimum avec cristal violet à 10 mg/L à 25 °C;
- (15) milieu minimum avec cristal violet à 50 mg/L à 25 °C;
- (16) milieu malt-sulfate de cuivre à 25 °C à la lumière, comprenant du malt extract à 10 g/L, du CuSO₄, 5 H₂O à 200 mg/L, de l'agar à 15 g/L)
- (17) milieu malt-sulfate de cuivre à 25 °C à l'obscurité, de composition identique à celle du milieu (16);
- (18) milieu Czapek-ammonium à 25 °C, comprenant du sucrose à 30 g/L, du NaNO₃ à 3 g/L, du K₂HPO₄ à 1 g/L, du MgSO₄, 7 H₂O à 0,5 g/L, du KCl à 0,5 g/L, du FeSO₄, 7 H₂O à 10 mg/L, du (NH₄)₂SO₄ à 5 g/L, de l'agar à 15 g/L);
- (19) milieu nitrito-saccharose à 25 °C, comprenant du saccharose à 10 g/L, du NaNO₂ à 2 g/L, de l'agar à 15 g/L;
- (20) milieu glycérol-nitrate à 25 °C, comprenant du glycérol à 5 ml/L, du NaNO₃ à 3g/L, de l'agar à 15 g/L;
Afin de déterminer leur profil biochimique, morphologique et physiologique;
f) Comparer le profil biochimique, morphologique et physiologique desdits champignons avec le profil biochimique, morphologique et physiologique spécifique de la souche *Trichoderma atroviride* selon la revendication 1; et
g) Isoler les champignons *Trichoderma atroviride* dont le profil biochimique, morphologique et physiologique correspond au profil biochimique, morphologique et physiologique spécifique de la souche *Trichoderma atroviride* selon la revendication 1.

## Patentansprüche

1. Ein *Trichoderma-atroviride*-Stamm, **dadurch gekennzeichnet, dass** er das folgende biochemische, morphologische und physiologische Profil aufweist:
| Kulturmedium | Wachstumsrate | Erscheinungsbild der Sporulation |
|---|---|---|
| (1) PDA-Referenzmedium bei 25 °C Kartoffel-Dextrose-Agar 39 g/l | sehr schnell - Auftreten eines transluzenten kurzen Thallus innerhalb von zwei Tagen | intensiv grün |
| Kulturmedium | Wachstumsrate in Bezug auf das PDA-Medium | Erscheinungsbild der Sporulation in Bezug auf das PDA-Medium |
|---|---|---|
| (2) Minimalmedium bei 4 °C 5 g/l Glucose + 5 g/l Pepton + MgSO₄, 0,5 g/l 7 H₂O + 1 g/l KH₂PO₄ + 25 mg/l Bengalrosa + 15 g/l Agar | sehr langsam | abwesend |
| (3) Minimalmedium bei 25 °C 5 g/l Glucose + 5 g/l Pepton + MgSO₄, 0,5 g/l 7 H₂O + 1 g/l KH₂PO₄+ 25 mg/l Bengalrosa + 15 g/l Agar | sehr geringfügig verlangsamt | intensiv grün bis beige |
| (4) Minimalmedium bei 37 °C 5 g/l Glucose + 5 g/l Pepton + MgSO₄, 0,5 g/l 7 H₂O + 1 g/l KH₂PO₄ + 25 mg/l Bengalrosa + 15 g/l Agar | kein Wachstum | keine Sporulation |
| (5) Minimalmedium 1 % Glucose bei 25 °C, wobei der Glucoseanteil von 5 g/l des Minimalmediums auf 10 g/l erhöht wird | verlangsamt | schwächer |
| (6) Minimalmedium 1 % Ethanol bei 25 °C, wobei der Glucoseanteil von 5 g/l des Minimalmediums durch Ethanol in einer Menge von 10 ml/l ersetzt wird | Myzel weiter ausgebreitet, aber weniger dicht | schwächer |
| (7) Minimalmedium 1 % Zitronensäure bei 25 °C, wobei der Glucoseanteil von 5 g/l des Minimalmediums durch Zitronensäure in einer Menge von 10 g/l ersetzt wird | Myzel weniger dicht | abwesend |
| (8) Minimalmedium 1 % Milchsäure bei 25 °C, wobei der Glucoseanteil von 5 g/l des Minimalmediums durch Milchsäure in einer Menge von 10 g/l ersetzt wird | geringfügig verlangsamt | schwächer |
| (9) Minimalmedium 0,2 % Glycin bei 25 °C, wobei der Peptonanteil von 5 g/l des Minimalmediums durch Glycin in einer Menge von 2 g/l ersetzt wird | geringfügig verlangsamt | schwächer |
| (10) Minimalmedium 0,2 % Ammoniumoxalat bei 25 °C, wobei der Peptonanteil von 5 g/l des Minimalmediums durch Ammoniumoxalat in einer Menge von 2 g/l ersetzt wird | geringfügig verlangsamt | unverändert |
| (11) Minimalmedium 1 % Ammoniumoxalat bei 25 °C, wobei der Peptonanteil von 5 g/l des Minimalmediums durch Ammoniumoxalat in einer Menge von 10 g/l ersetzt wird | sehr verlangsamt | abwesend |
| (12) Minimalmedium 0,2 % Harnstoff bei 25 °C, wobei der Peptonanteil von 5 g/l des Minimalmediums durch Harnstoff in einer Menge von 2 g/l ersetzt wird | Myzel weiter ausgebreitet, aber weniger dicht | schwächer |
| (13) Minimalmedium 0,2 % Natriumnitrit bei 25 °C, wobei der Peptonanteil von 5 g/l des Minimalmediums durch Natriumnitrit in einer Menge von 2 g/l ersetzt wird | Myzel weiter ausgebreitet, aber weniger dicht | unverändert |
| (14) Minimalmedium bei 10 mg/l Kristallviolett bei 25 °C | sehr verlangsamt | unverändert |
| (15) Minimalmedium bei 50 mg/l Kristallviolett bei 25 °C | sehr verlangsamt | abwesend |
| (16) Malz-Kupfersulfat-Medium bei 25 °C bei Licht 10 g/l Malzextrakt + CuSO₄, 200 mg/l 5 H₂O + 15 g/l Agar | Myzel weniger dicht | unverändert |
| (17) Malz-Kupfersulfat-Medium bei 25 °C bei Dunkelheit 10 g/l Malzextrakt + CuSO₄, 200 mg/l 5 H₂O + 15 g/l Agar | Myzel weniger dicht | unverändert |
| (18) Czapek-Ammonium-Medium bei 25 °C 30 g/l Sucrose + 3 g/l NaNO₃ + 1 g/l K₂HPO₄ + MgSO₄, 0,5 g/l 7 H₂O + 0,5 g/l KCl + FeSO₄, 10 mg/l 7 H₂O + 5 g/l (NH₄)₂SO₄ + 15 g/l Agar | Myzel weiter ausgebreitet, weniger dicht im zentralen Bereich | etwas schwächer |
| (19) Nitritosaccharose-Medium bei 25 °C 10 g/l Saccharose + 2 g/l NaNO₂ + 15 g/l Agar | Myzel viel weniger dicht | viel schwächer |
| (20) Glycerol-Nitrat-Medium bei 25 °C 5 ml/l Glycerol + 3 g/l NaNO₃ + 15 g/l Agar | Myzel viel weniger dicht | viel schwächer |
wobei dieser Stamm ferner die folgenden Kultur-Erscheinungsbilder aufweist:
- auf dem PDA(Kartoffel-Dextrose-Agar)-Medium tritt schnell, innerhalb von etwa zwei Tagen, ein transluzenter kurzen Thallus auf, der am dritten Tag mit dem Auftreten von Konidienträgern weiß wird; dieses kurze Myzel verfärbt sich nach fünf Tagen in ein sehr dunkles Grün, was ein Anzeichen einer intensiven Sporulation ist, und die Rückseite der Kultur ist farblos;
- auf dem MA(Malz-Agar)-Medium ist seine Entwicklung leicht anders und charakteristisch für die Spezies *Trichoderma atroviride,* das Myzel bildet konzentrische Sporulationskreise von intensivem Grün um den Inokulationspunkt herum mit einem Thallus, der bis zum Ende der Kultur transluzent bleibt; das Erscheinungsbild unter dem Mikroskop ist auf beiden Medien gleich, die Konidienträger sind stark verzweigt, wobei konidiogene Zellen (Phialiden) in Wirteln zu zweit oder dritt angeordnet sind; die Phialiden sind ampullenförmig, gerade oder gekrümmt, mit Abmessungen von 2,5 bis 3 µm mal 8 bis 14 µm und die Konidien, die an der Spitze der Phialiden verbunden sind, sind kugelig mit glatter Wand, grün und weisen einen Durchmesser von ungefähr 3 µm auf;
- der Stamm wächst zwischen 12 °C und 36 °C, mit einem optimalen Wachstum zwischen 20 °C und 25 °C, auf herkömmlichen Agarmedien vom Typ PDA, MA, OA (Hafermehl-Agar);
wobei die internen Spacer-Nucleotidsequenzen, die mit 1 und 2 des Stamms transkribiert wurden, mit denen des *Trichoderma-atroviride-Stamms* ATCC 36042 identisch sind.

2. Ein Kulturmedium, **dadurch gekennzeichnet, dass** es mindestens einen *Trichoderma atroviride* gemäß Anspruch 1 und mindestens ein Substrat, insbesondere ein Substrat pflanzlichen Ursprungs und insbesondere ein Substrat, das reich an Polysacchariden wie Stärke, Hemicellulose oder Cellulose ist, beinhaltet.

3. Kulturmedium gemäß Anspruch 2, **dadurch gekennzeichnet, dass** es mindestens ein inertes mineralisches Verdünnungsmittel beinhaltet.

4. Kulturmedium gemäß Anspruch 3, **dadurch gekennzeichnet, dass** das Verdünnungsmittel spezifisch ausgewählt ist zum Verdünnen und/oder Belüften des Kulturmediums, wobei das Verdünnungsmittel zum Beispiel ausgewählt ist aus der Gruppe, bestehend aus körnigen calcinierten Tonen wie Attapulgit, porösen Tonkugeln, Pulver aus vulkanischen Gesteinen wie Puzzolan oder Bimsstein, expandiertem Perlit, expandiertem Vermiculit und Steinwolle.

5. Kulturmedium gemäß einem der Ansprüche 2 bis 4, **dadurch gekennzeichnet, dass** es mindestens ein Mineralelement beinhaltet, das für das Wachstum des *Trichoderma-atroviride*-Stamms gemäß Anspruch 1 geeignet ist, wie ein Nitrat, Ammonium, ein Orthophosphat, Kalium, Magnesium, Calcium oder ein Spurenelement.

6. Kulturmedium gemäß einem der Ansprüche 2 bis 5, **dadurch gekennzeichnet, dass** es eine Säure, vorzugsweise eine Mineralsäure, beinhaltet, was ermöglicht, den pH-Wert des Mediums zwischen 2 und 4 und vorzugsweise zwischen 2,7 und 3,3 zu halten.

7. Ein dehydratisiertes Konzentrat von *Trichoderma atroviride* gemäß Anspruch 1, insbesondere in Form eines Pulvers.

8. Ein Verfahren zum Gewinnen eines Pulvers auf Basis von *Trichoderma atroviride* gemäß Anspruch 1, **dadurch gekennzeichnet, dass** es die Schritte des Fermentierens des Kulturmediums gemäß einem der Ansprüche 4 bis 6, des Dehydratisierens bei niedriger Temperatur, gefolgt von einem Schritt des Zerkleinerns, beinhaltet.

9. Eine Verwendung von *Trichoderma atroviride* gemäß Anspruch 1 als Stimulans der Keimung und/oder des Wachstums von Pflanzen.

10. Eine Verwendung von *Trichoderma atroviride* gemäß Anspruch 1 als biologisches Bekämpfungsmittel, insbesondere gegen pflanzenpathogene Pilze.

11. Ein Verfahren zum Isolieren eines *Trichoderma-atroviride-Stamms* gemäß Anspruch 1, **dadurch gekennzeichnet, dass** das Verfahren die folgenden aufeinanderfolgenden Schritte beinhaltet:
a) Entnehmen eines *Trichoderma-atroviride*-Pilzextrakts in einem geeigneten Medium, wie nicht kultiviertem oder kultiviertem Boden, an Pflanzen oder in verfaulenden Hölzern, die aus Reben, Ästen oder Baumstämmen stammen,
b) Verdünnen des entnommenen Extrakts mit sterilem Wasser,
c) Kultivieren dieses Extrakts auf mindestens einem selektiven Medium, wobei das selektive Medium in der Gruppe enthalten ist, die aus Folgendem besteht: MA2-Medium (2 % Malzagar) und RB-S-F-Medium (McFadden und Sutton),
d) Transplantieren der *Trichoderma-atroviride*-Pilze, die aus isolierten Sporen stammen, in Petrischalen, die PDA-Medium (Kartoffel-Dextrose-Agar) enthalten,
e) Kultivieren der transplantierten *Trichoderma-atroviride*-Pilze zwölf Tage lang unter den folgenden Wachstumsbedingungen:
- (1) PDA-Medium bei 25 °C, bei 39 g/l;
- (2) Minimalmedium bei 4 °C, beinhaltend 5 g/l Glucose, 5 g/l Pepton, MgSO₄, 0,5 g/l 7 H₂O, 1 g/l KH₂PO₄, 25 mg/l Bengalrosa, 15 g/l Agar;
- (3) Minimalmedium bei 25 °C, mit identischer Zusammensetzung wie das Minimalmedium (2);
- (4) Minimalmedium bei 37 °C, mit identischer Zusammensetzung wie das Minimalmedium (2);
- (5) Minimalmedium 1 % Glucose bei 25 °C, wobei der Glucoseanteil von 5 g/l des Minimalmediums auf 10 g/l erhöht wird;
- (6) Minimalmedium 1 % Ethanol bei 25 °C, wobei der Glucoseanteil von 5 g/l des Minimalmediums durch Ethanol in einer Menge von 10 ml/l ersetzt wird;
- (7) Minimalmedium 1 % Zitronensäure bei 25 °C, wobei der Glucoseanteil von 5 g/l des Minimalmediums durch Zitronensäure in einer Menge von 10 g/l ersetzt wird;
- (8) Minimalmedium 1 % Milchsäure bei 25 °C, wobei der Glucoseanteil von 5 g/l des Minimalmediums durch Milchsäure in einer Menge von 10 g/l ersetzt wird;
- (9) Minimalmedium 0,2 % Glycin bei 25 °C, wobei der Peptonanteil von 5 g/l des Minimalmediums durch Glycin in einer Menge von 2 g/l ersetzt wird;
- (10) Minimalmedium 0,2 % Ammoniumoxalat bei 25 °C, wobei der Peptonanteil von 5 g/l des Minimalmediums durch Ammoniumoxalat in einer Menge von 2 g/l ersetzt wird;
- (11) Minimalmedium 1 % Ammoniumoxalat bei 25 °C, wobei der Peptonanteil von 5 g/l des Minimalmediums durch Ammoniumoxalat in einer Menge von 10 g/l ersetzt wird;
- (12) Minimalmedium 0,2 % Harnstoff bei 25 °C, wobei der Peptonanteil von 5 g/l des Minimalmediums durch Harnstoff in einer Menge von 2 g/l ersetzt wird;
- (13) Minimalmedium 0,2 % Natriumnitrit bei 25 °C, wobei der Peptonanteil von 5 g/l des Minimalmediums durch Natriumnitrit in einer Menge von 2 g/l ersetzt wird;
- (14) 10 mg/l Minimalmedium mit Kristallviolett bei 25 °C;
- (15) 50 mg/l Minimalmedium mit Kristallviolett bei 25 °C;
- (16) Malz-Kupfersulfat-Medium bei 25 °C bei Licht, beinhaltend 10 g/l Malzextrakt, CuSO₄, 200 mg/l 5 H₂O, 15 g/l Agar;
- (17) Malz-Kupfersulfat-Medium bei 25 °C bei Dunkelheit, mit identischer Zusammensetzung wie das Medium (16);
- (18) Czapek-Ammoniummedium bei 25 °C, beinhaltend 30 g/l Sucrose, 3 g/l NaNO₃, 1 g/l K₂HPO₄, MgSO₄, 0,5 g/l 7 H₂O, 0,5 g/l KCl, FeSO₄, 10 mg/l 7 H₂O, 5 g/l (NH₄)₂SO₄, 15 g/l Agar;
- (19) Nitritosaccharose-Medium bei 25 °C, beinhaltend 10 g/l Saccharose, 2 g/l NaNO₂, 15 g/l Agar;
- (20) Glycerol-Nitrat-Medium bei 25 °C, beinhaltend 5 ml/l Glycerol, 3 g/l NaNO₃, 15 g/l Agar;
um ihr biochemisches, morphologisches und physiologisches Profil zu bestimmen;
f) Vergleichen des biochemischen, morphologischen und physiologischen Profils der Pilze mit dem spezifischen biochemischen, morphologischen und physiologischen Profil des *Trichoderma-atroviride-Stamms* gemäß Anspruch 1; und
g) Isolieren der *Trichoderma-atroviride*-Pilze, deren biochemisches, morphologisches und physiologisches Profil dem spezifischen biochemischen, morphologischen und physiologischen Profil des *Trichoderma-atroviride-Stamms* gemäß Anspruch 1 entspricht.

## Claims

1. A strain of *Trichoderma atroviride* **characterised in that** it possesses the following biochemical, morphological and physiological profile:
| Culture medium | Growth rate | Appearance of the sporulation |
|---|---|---|
| (1) PDA reference medium at 25°C 39 g/L potato dextrose agar | very fast - appearance of a translucent low thallus in around two days | deep green |
| Culture medium | Growth rate compared to the PDA medium | Appearance of the sporulation compared to the PDA medium |
|---|---|---|
| (2) minimum medium at 4°C 5 g/L glucose + 5 g/L peptone + 0.5 g/L MgSO₄. 7 H₂O + 1 g/L KH₂PO₄ + 25 mg/L rose bengal + 15 g/L agar | very slow | absent |
| (3) minimum medium at 25°C 5 g/L glucose + 5 g/L peptone + 0.5 g/L MgSO₄. 7 H₂O + 1 g/L KH₂PO₄ + 25 mg/L rose bengal + 15 g/L agar | very slightly slower | deep green to beige |
| (4) minimum medium at 37°C 5 g/L glucose + 5 g/L peptone + 0.5 g/L MgSO₄. 7 H₂O + 1 g/L KH₂PO₄ + 25 mg/L rose bengal + 15 g/L agar | no growth | no sporulation |
| (5) minimum medium 1% glucose at 25°C the 5 g/L glucose of the minimum medium is increased to 10 g/L | slower | weaker |
| (6) minimum medium 1% ethanol at 25°C the 5 g/L glucose of the minimum medium is replaced by 10 mL/L ethanol | mycelium more extensive but less dense | weaker |
| (7) minimum medium 1% citric acid at 25°C the 5 g/L glucose of the minimum medium is replaced by 10 g/L citric acid | mycelium less dense | absent |
| (8) minimum medium 1% lactic acid at 25°C the 5 g/L glucose of the minimum medium is replaced by 10 g/L lactic acid | slightly slower | weaker |
| (9) minimum medium 0.2% glycine at 25°C the 5 g/L peptone of the minimum medium is replaced by 2 g/L glycine | slightly slower | weaker |
| (10) minimum medium 0.2% ammonium oxalate at 25°C the 5 g/L peptone of the minimum medium is replaced by 2 g/L ammonium oxalate | slightly slower | unchanged |
| (11) minimum medium 1% ammonium oxalate at 25°C the 5 g/L peptone of the minimum medium is replaced by 10 g/L ammonium oxalate | much slower | absent |
| (12) minimum medium 0.2% urea at 25°C the 5 g/L peptone of the minimum medium is replaced by 2 g/L urea | mycelium more extensive but less dense | weaker |
| (13) minimum medium 0.2% sodium nitrite at 25°C the 5 g/L peptone of the minimum medium is replaced by 2 g/L sodium nitrite | mycelium more extensive but less dense | unchanged |
| (14) minimum medium with 10 mg/l crystal violet at 25°C | much slower | unchanged |
| (15) minimum medium with 50 mg/l crystal violet at 25°C | much slower | absent |
| (16) malt-copper sulfate medium at 25°C in light 10 g/L malt extract + 200 mg/L CuSO₄. 5 H₂O + 15 g/L agar | mycelium less dense | unchanged |
| (17) malt-copper sulfate medium at 25°C in darkness 10 g/L malt extract + 200 mg/L CuSO₄. 5 H₂O + 15 g/L agar | mycelium less dense | unchanged |
| (18) Czapek-ammonium medium at 25°C 30 g/L sucrose + 3 g/L NaNO₃ + 1 g/L K₂HPO₄ + 0.5 g/L MgSO₄. 7 H₂O + 0.5 g/L KCI + 10 mg/L FeSO₄. 7 H₂O + 5 g/L (NH₄)₂SO₄ + 15 g/L agar | mycelium more extensive less dense in the central area | a little weaker |
| (19) nitrite-saccharose medium at 25°C 10 g/L saccharose + 2 g/L NaNO₂ + 15 g/L agar | mycelium much less dense | much weaker |
| (20) glycerol-nitrate medium at 25°C 5 ml/L glycerol + 3 g/L NaNO₃ + 15 g/L agar | mycelium much less dense | much weaker |
said strain further possessing the following culture appearances:
- on PDA (Potato Dextrose Agar) medium, there quickly appears, in around two days, a translucent low thallus which becomes white on the third day with the appearance of the conidiophores; this low mycelium turns very dark green at the end of five days, indicating an intense sporulation and the underside of the culture is colourless;
- on MA (Malt Agar) medium, its development is slightly different and characteristic of the species *Trichoderma atroviride,* the mycelium forms concentric circles of intense green sporulation around the point of inoculation with a thallus which remains translucent until the end of the culturing; the microscopic appearance is the same on the two media, the conidiophores are highly branched with conidiogenous cells (phialides) arranged in whorls in 2s or 3s, said phialides are bulb-shaped, straight or curved, with dimensions of 2.5 to 3 µm by 8 to 14 µm and the conidia joined at the tip of the phialide, are globular with smooth walls, green in colour and measure around 3 µm in diameter;
- said strain develops between 12°C and 36°C with optimum growth between 20°C and 25°C on conventional agar media of the PDA, MA, OA (Oat Meal Agar) type;
the internal transcribed spacer nucleotide sequences 1 and 2 of said strain being identical to those of the strain *Trichoderma atroviride* ATCC 36042.

2. A culture medium **characterised in that** it comprises at least one *Trichoderma atroviride* according to claim 1 and at least one substrate, particularly a substrate of plant origin and more particularly a substrate which is rich in polysaccharides such as starch, hemicellulose or cellulose.

3. The culture medium according to claim 2 **characterised in that** it comprises at least one inert mineral diluent.

4. The culture medium according to claim 3 **characterised in that** said diluent is specifically selected to dilute and/or aerate said culture medium, for example, said diluent is chosen from the group constituted by calcined clays in grains such as attapulgite, blown clay beads, powders of volcanic rocks such as pozzolan or pumice stone, expanded perlite, expanded vermiculite and rock wool.

5. The culture medium according to any one of claims 2 to 4 **characterised in that** it comprises at least one mineral element useful for growing the *Trichoderma atroviride* strain according to claim 1, such as a nitrate, ammonium, an orthophosphate, potassium, magnesium, calcium or an oligo-element.

6. The culture medium according to any one of claims 2 to 5 **characterised in that** it comprises an acid, preferably a mineral acid, which makes it possible to keep the pH of the medium between 2 and 4 and preferably between 2.7 and 3.3.

7. A dehydrated concentrate of a *Trichoderma atroviride* according to claim 1, particularly in powder form.

8. A method of obtaining a powder based on *Trichoderma atroviride* according to claim 1, **characterised in that** it comprises the steps of fermenting the culture medium according to any one of claims 4 to 6, dehydrating at low temperature, followed by a crushing step.

9. The use of a *Trichoderma atroviride* according to claim 1 as a stimulant of plant germination and/or growth.

10. The use of a *Trichoderma atroviride* according to claim 1 as a biocontrol agent, notably against pathogenic plant fungi.

11. A method of isolating a *Trichoderma atroviride* strain according to claim 1, **characterised in that** said method comprises the following successive steps:
a) Sampling an extract of *Trichoderma atroviride* fungus in a suitable medium such as uncultivated or cultivated soil, on plants or in decaying wood from vines, branches or tree trunks,
b) Diluting the sampled extract with sterile water,
c) Culturing this extract on at least one selective medium, said selective medium being comprised in the group constituted by: the MA2 (malt agar 2%) medium and the RB-S-F medium (McFadden and Sutton),
d) Sub-culturing of the *Trichoderma atroviride* fungi from spores isolated in Petri dishes containing PDA (Potato Dextrose Agar) medium,
e) Culturing the sub-cultured *Trichoderma atroviride* fungi for twelve days in the following culturing conditions:
- (1) 39 g/L PDA medium at 25°C;
- (2) minimum medium at 4°C, comprising 5 g/L glucose, 5 g/L peptone, 0.5 g/L MgSO₄. 7 H₂O, 1 g/L KH₂PO₄, 25 mg/L rose bengal, 15 g/L agar;
- (3) minimum medium at 25°C, composition identical to that of minimum medium (2);
- (4) minimum medium at 37°C, composition identical to that of minimum medium (2);
- (5) minimum medium 1% glucose at 25°C, the 5 g/L glucose of the minimum medium being increased to 10 g/L;
- (6) minimum medium 1% ethanol at 25°C, the 5 g/L glucose of the minimum medium being replaced by 10 mL/L ethanol;
- (7) minimum medium 1% citric acid at 25°C, the 5 g/L glucose of the minimum medium being replaced by 10 g/L citric acid;
- (8) minimum medium 1% lactic acid at 25°C, the 5 g/L glucose of the minimum medium being replaced by 10 g/L lactic acid;
- (9) minimum medium 0.2% glycine at 25°C, the 5 g/L peptone of the minimum medium being replaced by 2 g/L glycine;
- (10) minimum medium 0.2% ammonium oxalate at 25°C, the 5 g/L peptone of the minimum medium being replaced by 2 g/L ammonium oxalate;
- (11) minimum medium 1% ammonium oxalate at 25°C, the 5 g/L peptone of the minimum medium being replaced by 10 g/L ammonium oxalate;
- (12) minimum medium 0.2% urea at 25°C, the 5 g/L peptone of the minimum medium being replaced by 2 g/L urea;
- (13) minimum medium 0.2% sodium nitrite at 25°C, the 5 g/L peptone of the minimum medium being replaced by 2 g/L sodium nitrite;
- (14) minimum medium with 10mg/L crystal violet at 25°C;
- (15) minimum medium with 50mg/L crystal violet at 25°C;
- (16) malt-copper sulfate medium at 25°C in light, comprising 10 g/L malt extract, 200 mg/L CuSO₄. 5 H₂O, 15 g/L agar;
- (17) malt-copper sulfate medium at 25°C in darkness, of identical composition to that of medium (16);
- (18) Czapek-ammonium medium at 25°C, comprising 30 g/L sucrose, 3 g/L NaNO₃, 1 g/L K₂HPO₄, 0.5 g/L MgSO₄. 7 H₂O, 0.5 g/L KCI, 10 mg/L FeSO₄. 7 H₂O, 5 g/L (NH₄)₂SO₄, 15 g/L agar;
- (19) nitrite-saccharose medium at 25°C, comprising 10 g/L saccharose, 2 g/L NaNO₂, 15 g/L agar;
- (20) glycerol-nitrate medium at 25°C, comprising 5 ml/L glycerol, 3 g/L NaNO₃, 15 g/L agar;
In order to determine their biochemical, morphological and physiological profile;
f) Comparing the biochemical, morphological and physiological profile of said fungi to the specific biochemical, morphological and physiological profile of the *Trichoderma atroviride* strain according to claim 1; and
g) Isolating the *Trichoderma atroviride* fungi whose biochemical, morphological and physiological profile corresponds to the specific biochemical, morphological and physiological profile of the *Trichoderma atroviride* strain according to claim 1.
